Europäisches Patentamt

European Patent Office

Office européen des brevets

(11) Publication number: **0 640 688 A1**

# EUROPEAN PATENT APPLICATION

(21) Application number: **94107862.8**

(22) Date of filing: **14.12.88**

(51) Int. Cl.6: **C12N 15/11**, C12N 9/00, C07H 21/02, A61K 31/70

This application was filed on 20.05.1994 as a divisional application to the application mentioned under INID code 60.

(30) Priority: **15.12.87 AU PI5911/87**
**19.08.88 AU PI9950/88**
**09.09.88 AU PJ0353/88**
**04.11.88 AU PJ1304/88**
**07.11.88 AU PJ1333/88**

(43) Date of publication of application:
**01.03.95 Bulletin 95/09**

(60) Publication number of the earlier application in accordance with Art.76 EPC: **0 321 201**

(84) Designated Contracting States:
**AT BE CH DE ES FR GB GR IT LI LU NL SE**

(71) Applicant: **GENE SHEARS PTY. LIMITED**
**c/o Macphillamy Cummins & Gibson**
**10th Floor, National Mutual Centre**
**Darwin Place**
**Canberra City (AU)**

(72) Inventor: **Gerlach,Wayne Lyle**
**22 Goble Street,**
**Hughes,**
**Australian Capital Territory ,2605, (AU)**
Inventor: **Haseloff,James Phillip**
**17 Hemmant Street,**
**O'Connor,**
**Australian Capital Territory,2601 (AU)**
Inventor: **Jennings,Philip Anthony**
**13 Lobelia Street,**
**West Chatswood,**
**New South Wales,2066 (AU)**
Inventor: **Cameron,Fiona Helen**
**No.2 Rounce Avenue,**
**Forestville,**
**New South Wales,2087 (AU)**

(74) Representative: **Feiler, Lothar (DE)**
**Patentanwälte**
**Henkel, Feiler, Hänzel & Partner**
**Möhlstrasse 37**
**D-81675 München (DE)**

(54) **Ribozymes.**

(57) Synthetic RNA molecules having highly specific endoribonuclease activity (hereinafter referred to as "ribozymes") are described. The ribozymes comprise a hybridizing region which is complementary in nucleotide sequence to at least part of a target RNA, and a catalytic region which is adapted to cleave the target RNA.
Methods for inactivating RNA, and compositions containing ribozymes are also described.

FIG.3.

The present invention relates to a class of synthetic RNA molecules and derivatives thereof, hereinafter referred to as ribozymes, which possess highly specific endoribonuclease activity.

A number of naturally occurring RNA molecules such as avocardo sunblotch viroid (ASBV), the satellite RNAs of tobacco ringspot virus (sTobRV) and lucerne transient streak virus (sLTSV), undergo self-catalysed cleavage. Such cleavage appears to be an essential and unique part of the life cycle of these and other RNAs.

Self-catalysed RNA cleavage reactions share a common requirement for divalent metal ions and neutral or higher pH, and result in the production of RNA with termini possessing 5' hydroxyl and 2',3' cyclic phosphate groups (Prody et al., Science 231: 1577-1580 (1986) and Buzayan et al., Virology 151: 186-199 (1986)). The cleavage reactions are catalysed by the RNAs themselves, presumably as a result of conformation bringing reactive groups into close proximity. The sites of self-catalysed cleavage in naturally occurring RNAs are located within highly conserved regions of RNA secondary structure (Buzayan et al., Proc. Natl. Acad. Sci. USA 83: 8859-8862 (1986) and Forster, A.C. and Symons, R.H. Cell 50: 9-16 (1987)).

Experiments we have carried out on the satellite RNAs of tobacco ringspot virus (sTobRV) have led to the design of novel endoribonucleases (hereinafter referred to as "ribozymes"), that is, enzymes comprised of RNA which catalyse specific cleavage of RNA target molecules.

The term ribozyme as used in the specification refers to molecules comprised wholly of RNA or derivatives thereof.

The ribozymes of the present invention are distinct from the RNA endoribonuclease which occurs naturally in Tetrahymena Fhermophila (known as the IVS, or L-19 IVS RNA) and which has been extensively described by Thomas Cech and collaborators (Zaug, A.J. et al, Science (1984) 224: 574-578; Zaug, A.J. and Cech, T.R., Science (1986) 231: 470-475; Zaug, A.J., et al, Nature (1986) 324: 429-433; published International patent application No. WO 88/04300 by University Patents Inc.). The Cech endoribonuclease has an eight base pair active site which hybridizes to a target RNA sequence whereafter cleavage of the target RNA takes place, with a requirement for free guanosine or guanosine derivatives. The fragments which arise from cleavage contain terminal 5' phosphate and 3' hydroxyl groups. The limited number of nucleotides available for hybridization to an RNA substrate limits the effectiveness or efficiency of the Cech endoribonuclease as in general, oligonucleotides comprising less than twelve nucleotides hybridize poorly to target sequences. It also appears that a number of nucleotides in the active site of the Cech endoribonuclease may need to be conserved for efficient endoribonuclease activity. This restricts the number of permutations of active site sequences which can be engineered to effect hybridization to target sequences, thereby restricting the range of RNA target sequences cleavable by the Cech endoribonuclease. The Cech endoribonuclease also modifies RNA by adding a free guanosine nucleotide to the 5' end of cleaved RNA.

In contrast, the ribozymes of the present invention hybridize efficiently to a wide range of target RNA sequences, and do not modify the cleaved target RNA.

The ribozymes of the present invention comprise a hybridizing region which is complementary in nucleotide sequence to at least part of a target RNA, and a catalytic region which is adapted to cleave the target RNA. The hybridizing region contains 9 or more nucleotides.

In a preferred aspect the ribozymes of the present invention have a hybridizing region comprising one or more arms formed of single stranded RNA and having a sequence complementary to at least part of a target RNA, said one or more arms being associated with a catalytic region capable of cleaving said target RNA; and where the hybridizing region comprises a single arm of RNA said arm contains at least 9 nucleotides, and where the hybridizing region comprises 2 or more arms of RNA, the sum of nucleotides in said arms is greater than 9 nucleotides.

In one embodiment of the invention, there is provided a ribozyme of the formula 1:

```
            3'                        5'
              (X) X           X(X)
                 n               n'
              (I)    A         C      (I)
                     A         U
                     G    1A   G          (II)              (1)
                     C *  G G  A
                     |    |  X
                     X'   X"
              5'        3'
```

wherein;

    X represents any ribonucleotide and each X residue may be the same or different;

    the sum of n and n' is greater than 6 and n and n' may be the same or different;

    an (*) represents a base pair between complementary ribonucleotides;

    X' and X" represent oligoribonucleotides of complementary sequence along at least part of their length to allow base pairing between the oligoribonucleotides, or X' and X" together form a single RNA sequence wherein at least part of said sequence comprises a stem formed by base pairing between complementary nucleotides; and

    optionally, an additional nucleotide selected from one of A, G, C or U may be inserted after $^1$A in formula (1).

    Region (I) of formula (1) represents the arms or flanking sequences of a ribozyme which hybridize to respective portions of a target RNA sequence. The arms may hybridize along the full length of the target RNA or part thereof. An RNA catalytic region is depicted at region (II) of formula 1. The catalytic region may contain one or more additional nucleotides which do not adversely effect catalytic activity. Such additions could be readily tested for ribozyme activity without undue experimentation following the teachings of the specification. The catalytic region may also form part of the hybridizing region.

    The oligoribonucleotides X' and X" may comprise up to 5,000 or more nucleotides.

    According to a further specific embodiment of the present invention there is provided a ribozyme of the formula 2:

```
            3'                          5'
              (X) A             X(X)
                 n                  n'
                      A           C
                      A             U
                      G       1A   G
                      C  *  G G   A              (2)
                      X  *  X  X
                      X  *  X
                     (X)  *  (X)
                        m      m'
                      X        X
                        B
```

wherein;

    X represents any ribonucleotide and each X residue may be the same or different;

    an (*) represents a base pair between complementary ribonucleotides;

    n and n' are as previously defined;

m and m' are 1 or more and may be the same or different;

B represents a bond, a base pair, a ribonucleotide, or an oligoribonucleotide containing at least 2 ribonucleotides;

and optionally, an additional nucleotide selected from any one of A, G, C or U may be inserted after $^I$A in formula (2).

The ribozymes of the present invention may be prepared by methods known per se in the art for the synthesis of RNA molecules. (For example, according to recommended protocols of Promega, Madison, WI, USA). In particular, the ribozymes of the invention may be prepared from a corresponding DNA sequence (DNA which on transcription yields a ribozyme, and which may be synthesized according to methods known per se in the art for the synthesis of DNA) operably linked to an RNA polymerase promoter such as a promoter for T7 RNA polymerase or SP6 RNA polymerase. A DNA sequence corresponding to a ribozyme of the present invention may be ligated into a DNA transfer vector, such as plasmid or bacteriophage DNA. Where the transfer vector contains an RNA polymerase promoter operably linked to DNA corresponding to a ribozyme, the ribozyme may be conveniently produced upon incubation with an RNA polymerase. Ribozymes may, therefore, be produced in vitro by incubation of RNA polymerase with an RNA polymerase promoter operably linked to DNA corresponding to a ribozyme, in the presence of ribonucleotides. In vivo, prokaryotic or eukaryotic cells (including mammalian and plant cells) may be transfected with an appropriate transfer vector containing genetic material corresponding to a ribozyme in accordance with the present invention, operably linked to an RNA polymerase promoter such that the ribozyme is transcribed in the host cell. Transfer vectors may be bacterial plasmids or viral RNA or DNA. Nucleotide sequences corresponding to ribozymes are generally placed under the control of strong promoters such as, for example, the lac, SV40 late, SV40 early, metallothionin, or λ promoters. Ribozymes may be directly transcribed in-vivo from a transfer vector, or alternatively, may be transcribed as part of a larger RNA molecule. For example, DNA corresponding to ribozyme sequences may be ligated into the 3' end of a carrier gene, for example, after a translation stop signal. Larger RNA molecules may help to stabilize the ribozyme molecules against nuclease digestion within cells. On translation the carrier gene may give rise to a protein, whose presence can be directly assayed, for example, by enzymatic reaction. The carrier gene may, for example, encode an enzyme.

In a further aspect of the invention, there is provided a DNA transfer vector which contains a DNA sequence corresponding to a ribozyme operably linked to a promoter to provide transcription of the ribozyme.

In one preferred method of producing a ribozyme, two synthetic oligonucleotides of complementary sequence are prepared by standard procedures (for example, using an Applied Biosystems Model 380A DNA Synthesizer (Applied Biosystems Inc., Foster City, California 94404)), and hybridized together. One of the oligonucleotides encodes a desired ribozyme. The respective ends of the hybridized oligonucleotides correspond to different restriction enzyme sites, say EcoRI at one end and PstI at the other end. After cleavage with appropriate restriction enzymes (EcoRI and PstI in the above example), the double stranded DNA fragment may be cloned into a transfer vector. Where the plasmid vector contains an RNA polymerase promoter upstream from the DNA sequence corresponding to a ribozyme of the present invention, RNA transcripts corresponding to the ribozyme can be conveniently prepared either in-vitro or in-vivo. Where the ribozyme is comprised of two halves held together by base-pairing between complementary nucleotides, each half of the ribozyme may be produced according to the above methods, and the halves incubated together to form the ribozyme.

The preferred ribozymes of the present invention cleave target RNA which contains the sequence X°UY, where X° is any ribonucleotide, U is uracil and Y is adenine, cytosine or uracil. X°U forms part of a base pair flanking region and Y is not base paired. Preferably, but by no means exclusively, X° is guanidine, and X°UY is GUC or GUA. Any RNA molecule containing these sequences can be cleaved with the ribozymes of the present invention. Once the sequence of an RNA transcript containing the sequence X°UY has been determined, the arms of the ribozyme sequence can be synthesised to be complementary to, and thus hybridizable to, the RNA on the target sequence flanking the X°UY sequence. On hybridization of the arms of the ribozyme to the target RNA sequence flanking the X°UY sequence, the catalytic region of the ribozyme cleaves the target RNA within the X°UY sequence. RNA cleavage is facilitated in the presence of magnesium or other divalent cation at a pH of approximately 8.0.

Accordingly, the preferred ribozymes of the present invention can be engineered to cleave any RNA whose sequence is known. The high frequency of the residues cleaved by the ribozymes in RNA (1:64 for GUC in an RNA with random and equal frequency of base distribution) means that a number of potential sites for ribozyme cleavage can be confidently predicted in any given target RNA.

According to another aspect of the present invention there is provided a method for the inactivation of a target RNA sequence, which comprises reacting said target RNA sequence with a ribozyme of the present invention.

In-vivo, that is, within the cell or cells of an organism, a transfer vector such as a bacterial plasmid or viral RNA or DNA, encoding one or more ribozymes, may be transfected into cells e.g. (Llewellyn et al., J. Mol. Biol. (1987) 195: 115-123; Hanahan et al. J. Mo. Biol (1983) 166). Once inside the cell, the transfer vector may replicate, and be transcribed by cellular polymerases to produce ribozyme RNAs which then inactivate a desired target RNA. Alternatively, a transfer vector containing one or more ribozyme sequences may be transfected into cells or introduced into cells by way of micromanipulation techniques such as microinjection, such that the transfer vector or a part thereof becomes integrated into the genome of the host cell. Transcription of the integrated genetic material gives rise to ribozymes, which act to inactivate a desired target RNA.

The ribozymes of the present invention have extensive therapeutic and biological applications. For example, disease causing viruses in man and animals may be inactivated by administering to a subject infected with a virus, a ribozyme in accordance with the present invention adapted to hybridize to and cleave RNA transcripts of the virus. Such ribozymes may be delivered by parenteral or other means of administration. Alternatively, a subject infected with a disease causing virus may be administered a non-virulent virus such as vaccinia or adenovirus which has been genetically engineered to contain DNA corresponding to a ribozyme operably linked to an RNA promoter, such that the ribozyme is transcribed in the cells of the host animal, transfected with the engineered virus, to effect cleavage and/or inactivation of the target RNA transcript of the disease causing virus. The ribozymes of the present invention have particular application to viral diseases caused for example, by the herpes simplex virus (HSV) or the AIDS virus (HIV).

The ribozymes of the present invention also have particular application to the inactivation of RNA transcripts in bacteria and other prokaryotic cells, plants and animals. In bacteria, RNA transcripts of, for example,bacteriophage, which cause bacterial cell death, may be inactivated by transfecting a cell with a DNA transfer vector which is capable of producing a ribozyme in accordance with the present invention which inactivates the phage DNA. Alternatively, the ribozyme itself may be added to and taken up by the bacterial cell to effect cleavage of the phage RNA.

RNA transcripts in plants may be inactivated using ribozymes encoded by a transfer vector such as the Ti plasmid of <u>Agrobacterium</u> <u>tumefaciens</u>. When such vectors are transfected into a plant cell, the ribozymes are produced under the action of RNA polymerase and may effect cleavage of a specific target RNA sequence. Accordingly, plant viruses whose RNA sequence are known, or the RNA transcripts of plant genes, may be inactivated using ribozymes.

Endogenous gene transcripts in plants, animals or other cell types may be inactivated using the ribozymes of the present invention. Accordingly, undesirable phenotypes or characteristics may be modulated. It may, for example, be possible using the ribozymes of the present invention to remove stones from fruit or treat hereditry diseases in humans which are caused by the production of a deleterious protein, or over production of a particular protein.

The present invention will now be illustrated by way of non-limiting example only, with reference to the following non-limiting Examples, and Figures.

In the Figures:

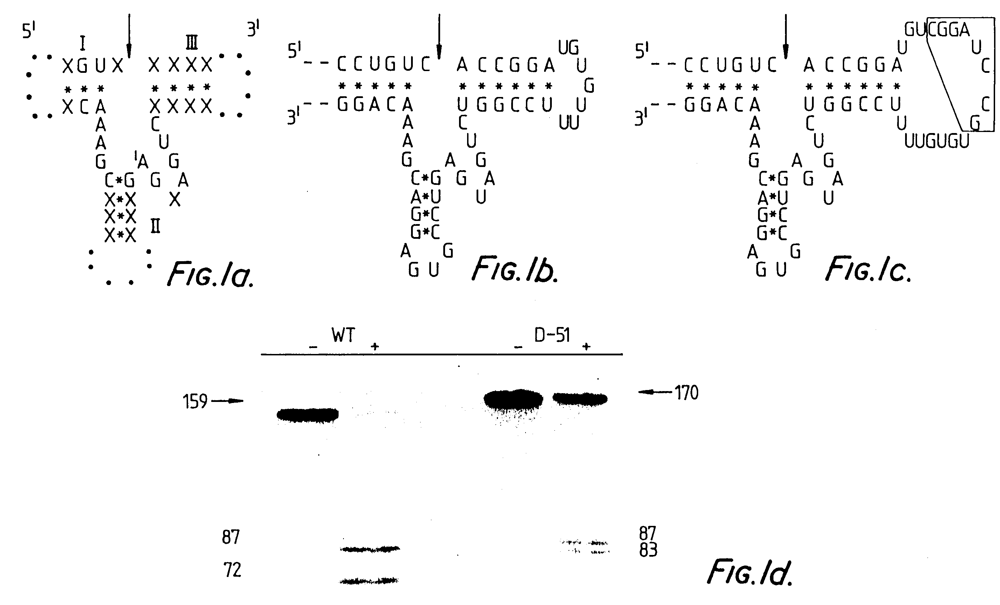

FIGURE 1 shows RNA self cleavage sites of wild type and mutated RNAs; and an electrophoretic profile showing self-catalysed RNA cleavage products.

(a) Summarises the conserved structures associated with naturally-occurring RNA cleavage sites in ASBV, newt satellite DNA transcripts and the satellite RNAs of sTobRV, LTSV, velvet tobacco mottle virus, solanum nodiflorum mottle virus and subterranean clover mottle virus. Nucleotide sequences which are conserved between these structures are shown, while others are represented as X. Base-pairing is represented by "*" and the site for RNA cleavage is arrowed.

(b) Shows the conserved nucleotide sequence associated with the cleavage of the (+) strand of sTobRV RNA. The cleavage site is arrowed.

(c) An in vitro mutant of sTobRV containing an insertion of eight nucleotides (shown boxed) together with a flanking duplication of three nucleotides (UGU residues 7 to 9) is shown.

(d) Sub-cloned HaeIII fragments of wild type sTobRV and the D-51 in vitro mutant were each transcribed in both the (+) and (-) orientations and radiolabelled transcripts fractionated by polyacylamide gel electrophoresis. The positions of uncleaved 159 and 170 base transcripts from the wild type (WT) and mutant (D-51) sequences are arrowed; sizes of cleavage products are shown.

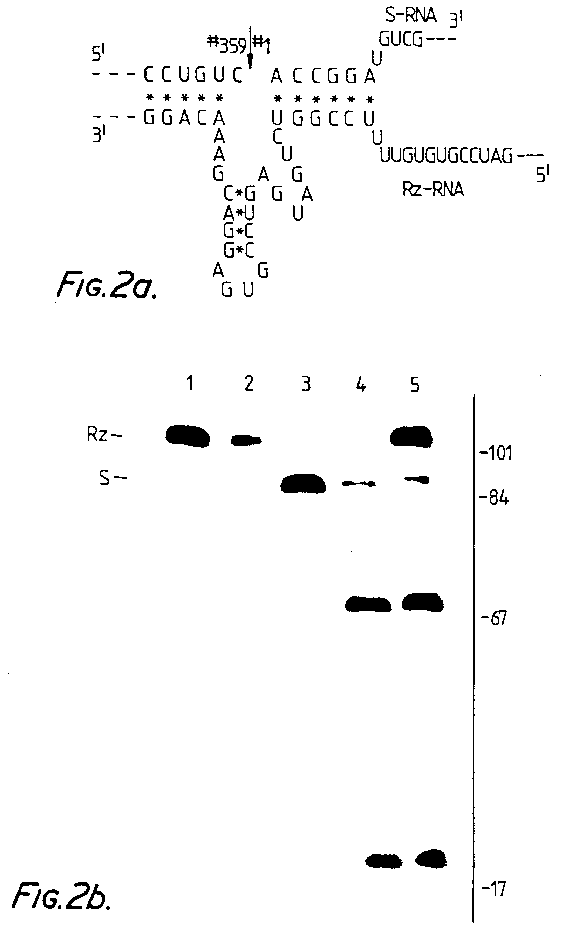

FIGURE 2 shows the nucleotide sequence of a ribozyme and the products of ribozyme cleavage separated by gel electrophoresis.

(a) The inserted nucleotides in the D-51 mutant (FIG. 1c) contains a BamHI restriction endonuclease site. BamHI was used to split the mutant DNA, and the two sequences were sub-cloned and transcribed separately in vitro. The RNA transcripts are shown schematically, with potential base-pairings between the RNAs indicated "*". The fragment containing the arrowed site for cleavage is referred to as S-RNA, the fragment containing the ribozyme is designated Rz-RNA.

(b) [$^{32}$P]-Rz-RNA (101 bases) was incubated alone (lane 1), and with unlabelled S-RNA (lane 2). [$^{32}$P]-S-RNA was incubated alone (lane 3), and with unlabelled and $^{32}$P labelled Rz-RNAs (lanes 4 & 5 respectively).

FIGURE 3 shows a schematic model of a ribozyme according to one embodiment of the present invention. Region A represents the cleavage sequence within the target RNA. Region B represents a catalytic region, and regions C represent the arms of the ribozyme.

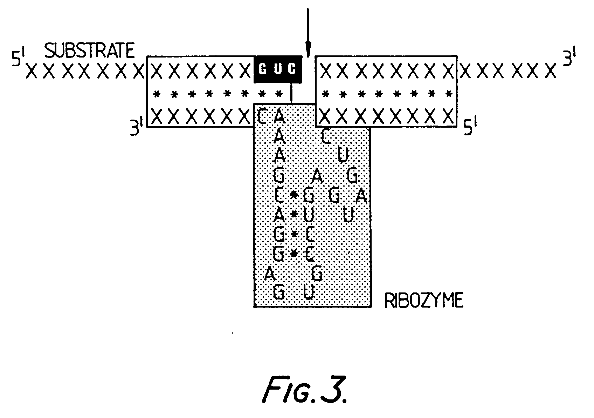

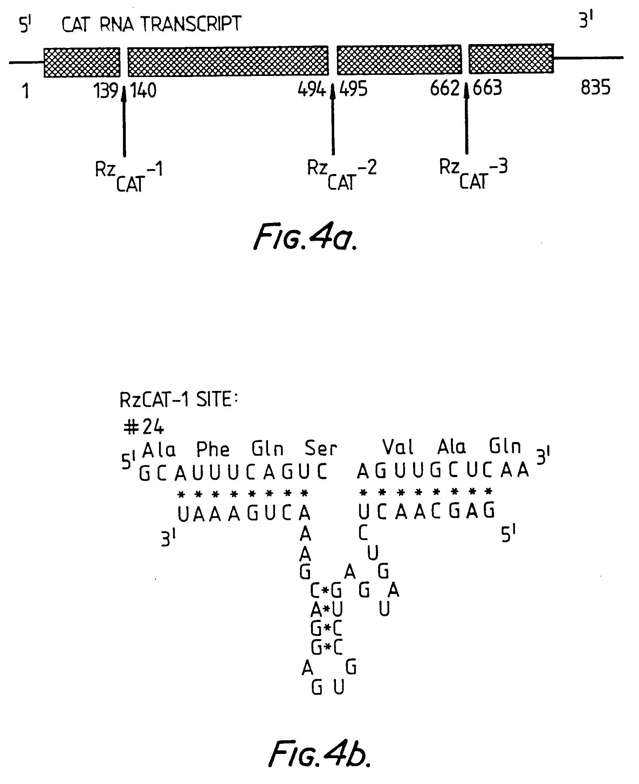

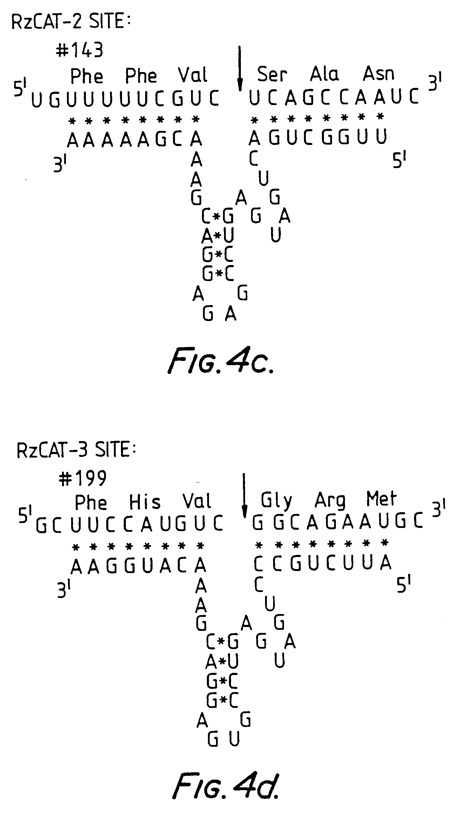

FIGURE 4 shows the design of ribozymes targeted against the CAT (chloramphenicol acetyl transferase) gene transcript. Ribozymes, termed RzCAT-1, 2 and 3, were targeted against three sites within an 835 base in vitro transcript of the CAT gene. The relative locations of the cleavage sites on the transcript are shown schematically with the flanking bases numbered (a). The three ribozyme sequences are shown (-(b) to (d)) with their target sequences. Amino-acid sequences of the CAT gene are numbered and the predicted sites for RNA cleavage arrowed. RzCAT-1 and 3 contain 24 base sequences derived from (+) strand sTobRV (region B, FIG. 3), while RzCAT-2 contains a single U-A change in this region.

FIGURE 5 shows the results of CAT RNA cleavage with ribozymes RzCAT-1 to 3.

(a) The [$^{32}$P]-CAT RNAs were gel fractionated after incubation alone (-) or with with one of the three ribozymes, RzCAT-1 to 3 (lanes 1, 2 & 3 respectively). The location of full-length transcript is shown arrowed.

(b) 5' Terminal base analysis. The 3' fragments produced by ribozyme cleavage of CAT mRNA were [5'-$^{32}$P]-kinased, gel purified, subjected to complete nuclease digestion and the released terminal residues fractionated by pH 3.5 polyacrylamide gel electrophoresis. The 5' terminal nucleotides, determined by reference to markers (lane M), were A, U and G for the fragments produced by RzCAT-1 to 3 (lanes 1,2 and 3 respectively).

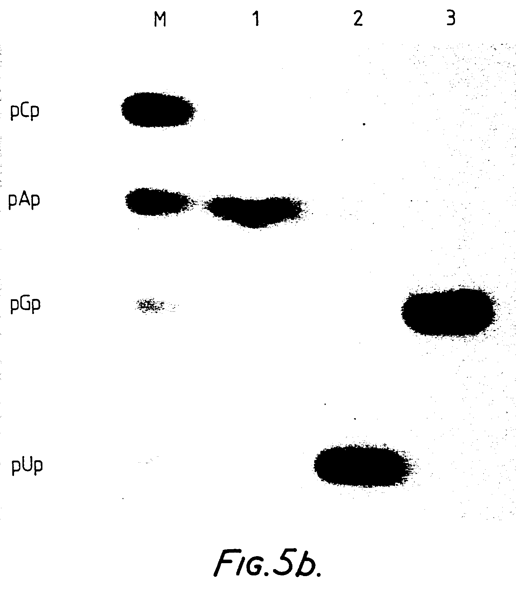

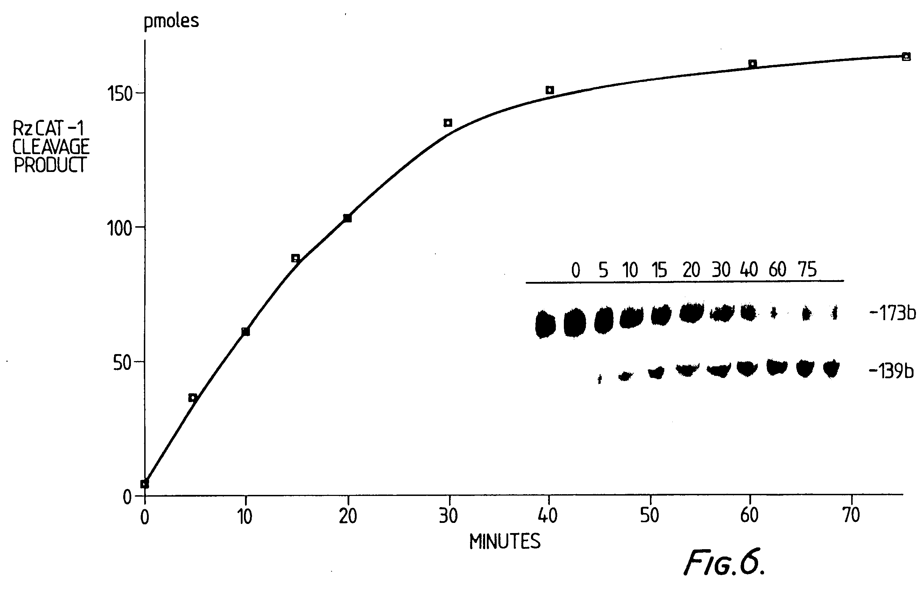

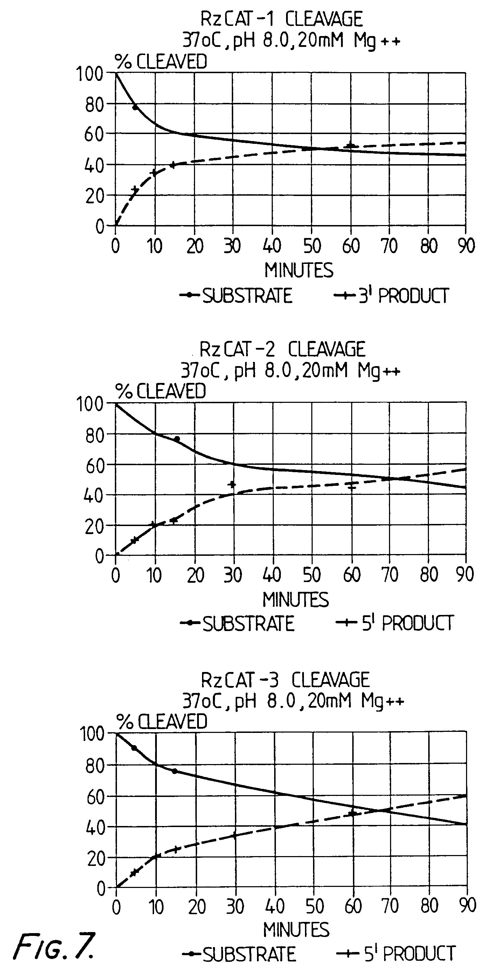

FIGURE 6 shows a time course of ribozyme (RzCAT-1) catalytic activity against CAT RNA. The amounts of the 139 nucleotide cleavage product were quantified and plotted. The inset shows the accumulation of the 139 base fragment with time, after polyacrylamide gel electrophoresis.

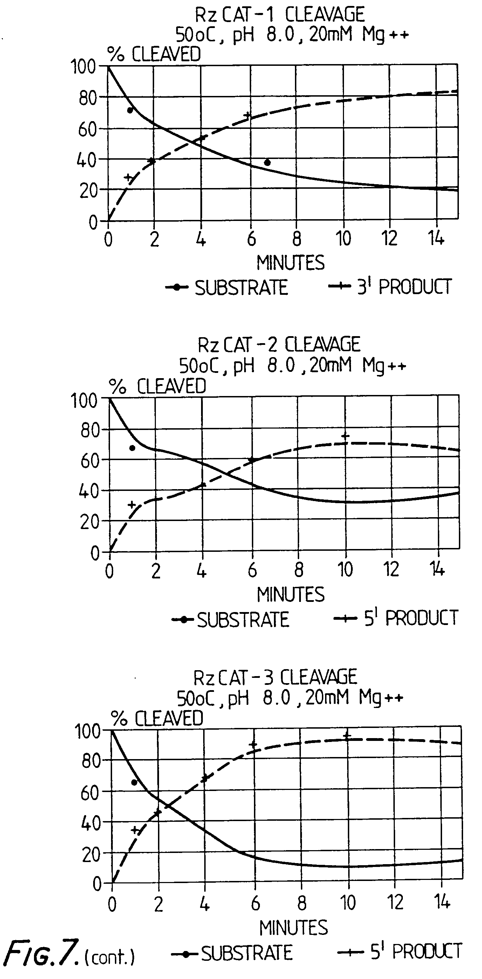

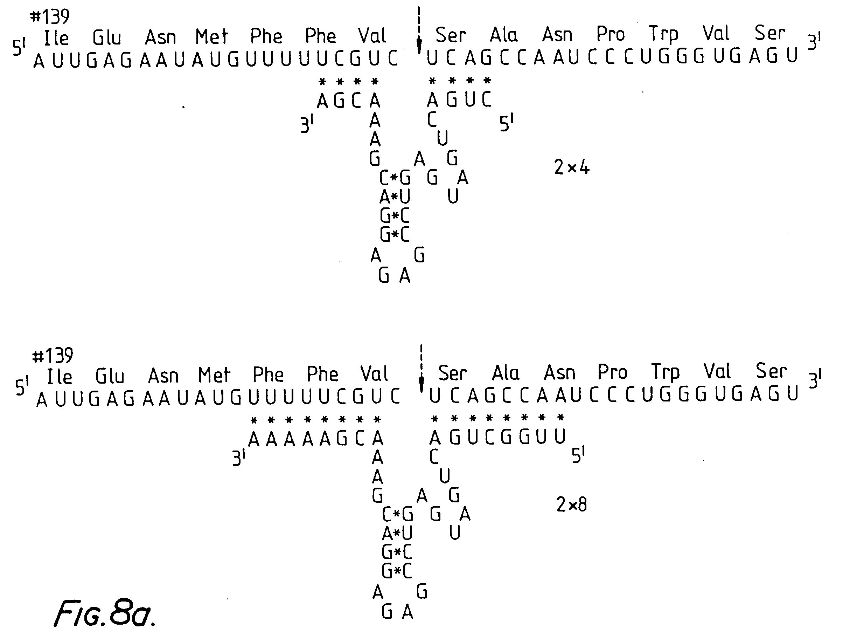

FIGURE 7 shows the relative rates of cleavage of CAT RNA under different temperature conditions. Substrate RNA is represented by a solid line. In each case cleavage product is represented by a broken line.

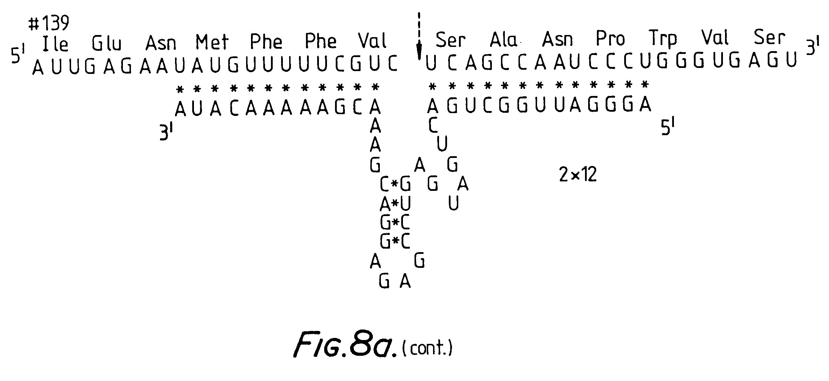

FIGURE 8 depicts three ribozymes (corresponding to RzCAT-2) having arms or flanking sequence of varying length.

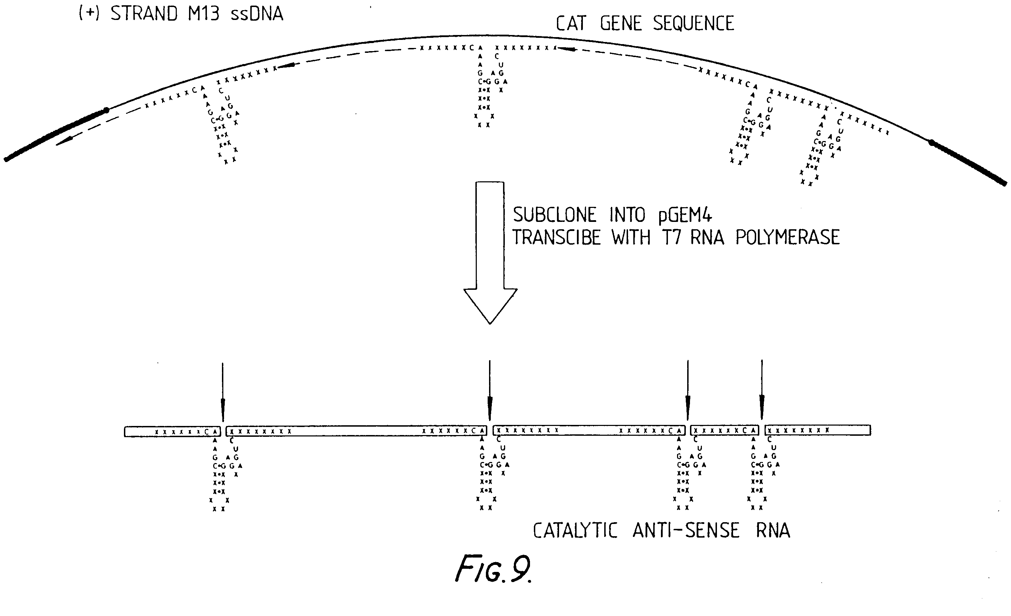

FIGURE 9 shows the scheme for production of a ribozyme comprising catalytic anti-sense RNA containing each of the cleavage domains RzCAT-1 to 3.

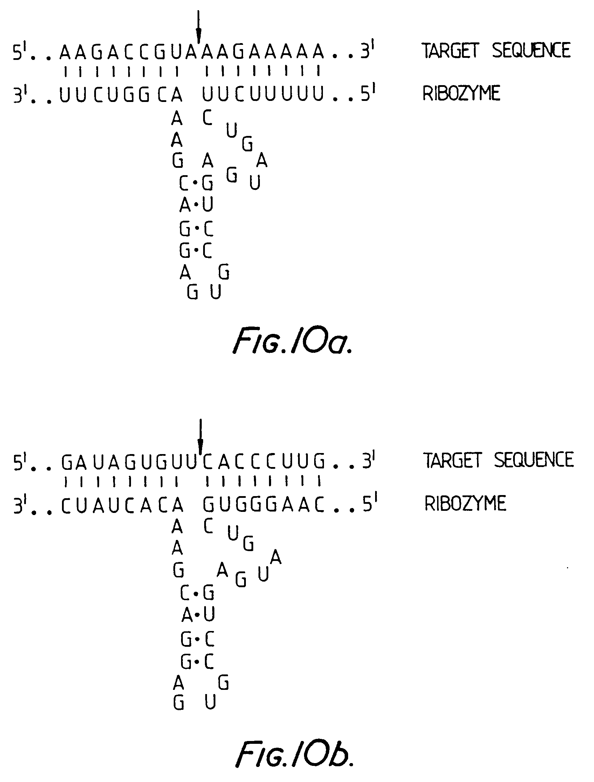

FIGURE 10 shows ribozymes hybridizing to a target sequences containing the GUA (10a) and GUU (10b) motifs in CAT mRNA.

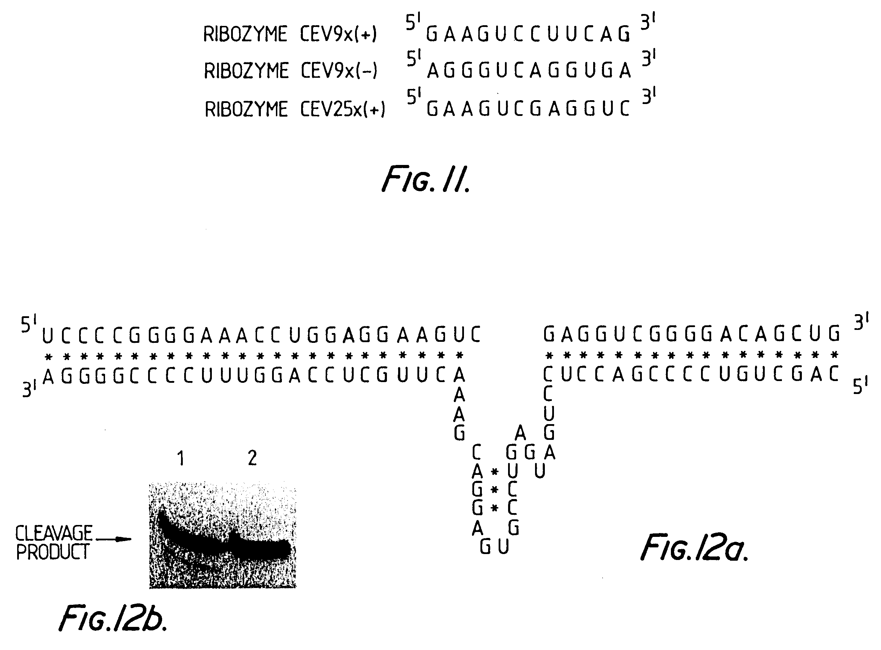

FIGURE 11 shows sites for self catalysed RNA cleavage in citrus exocortis viroid (CEV) RNA and its complement.

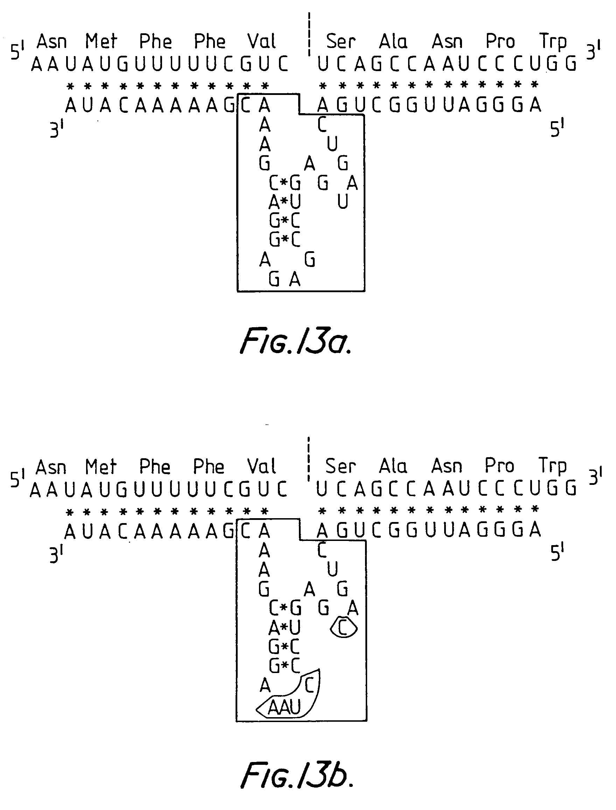

FIGURE 12 shows the ribozyme RzCEV25x(+) hybridized to target CEV RNA(a), and a gel electrophoretic profile of (+)CEV RNA and complementary (-) CEV RNA incubated with RzCEV25x(+) (b, lanes 1 and 2 respectively. Cleavage product is arrowed).

FIGURE 13 shows the ribozyme RzCAT-2 hybridizing to its target sequence (a) and ribozyme RzSCMoV(b). The catalytic domain in each ribozyme is boxed. Differences in the catalytic region of RzSCMoV, when compared with RzCAT-2 are marked.

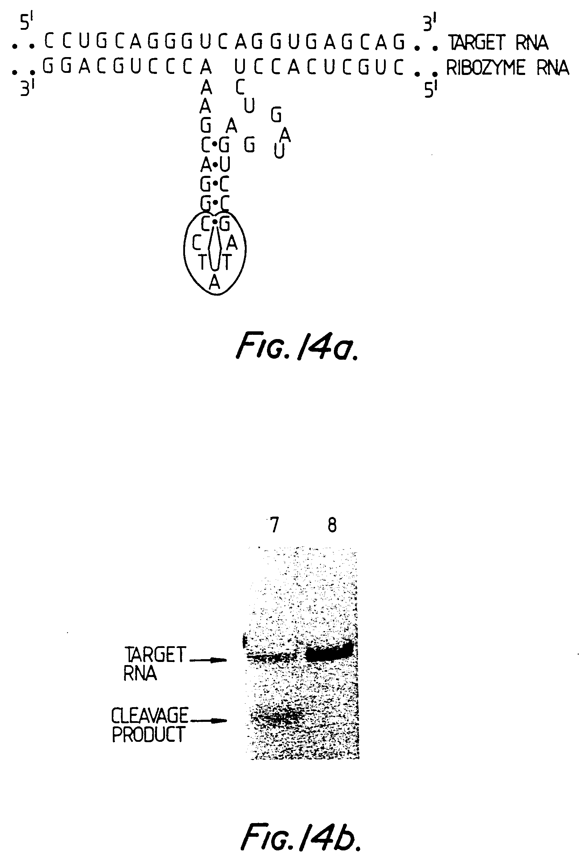

FIGURE 14 shows the ribozyme RzCEV-2 hybridizing to a target sequence in citrus exocortis viroid (CEV) RNA. The cleavage site corresponds to nucleotide-336 in the CEV RNA sequence. The alteration in nucleotide sequence in the catalytic domain, when compared with the catalytic domain of sTobRV, is circled (a). Fig 14(b) shows an electrophoretic profile of a control [(-) strand of CEV] RNA, lane 7 and the (+) strand of CEV RNA lane 8, after incubation with RzCEV2.

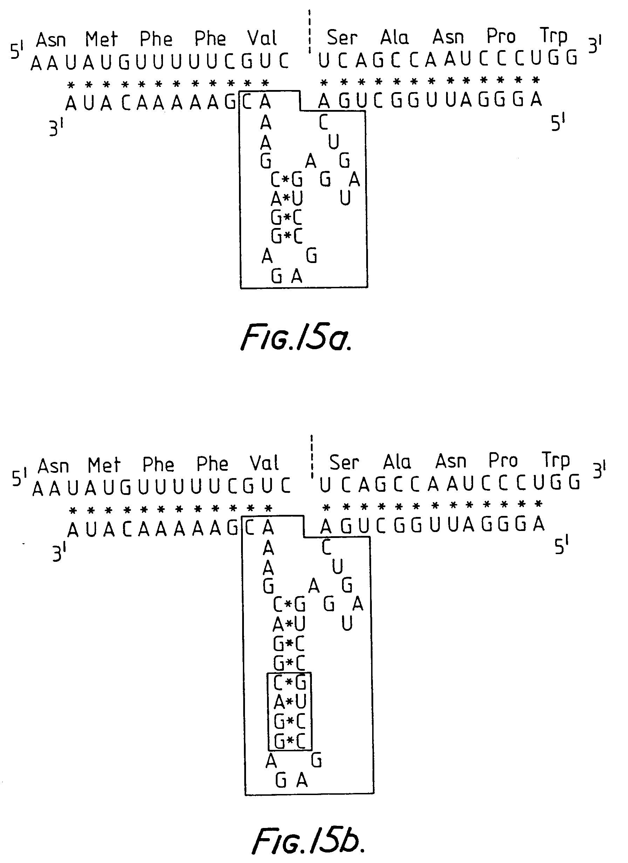

FIGURE 15 shows the ribozyme RzCAT-2 (a) compared with ribozyme RzCAT-2B (b). Catalytic domains are boxed. Changes is the catalytic domain of RzCAT-2B compared with RzCAT-2 are also boxed;

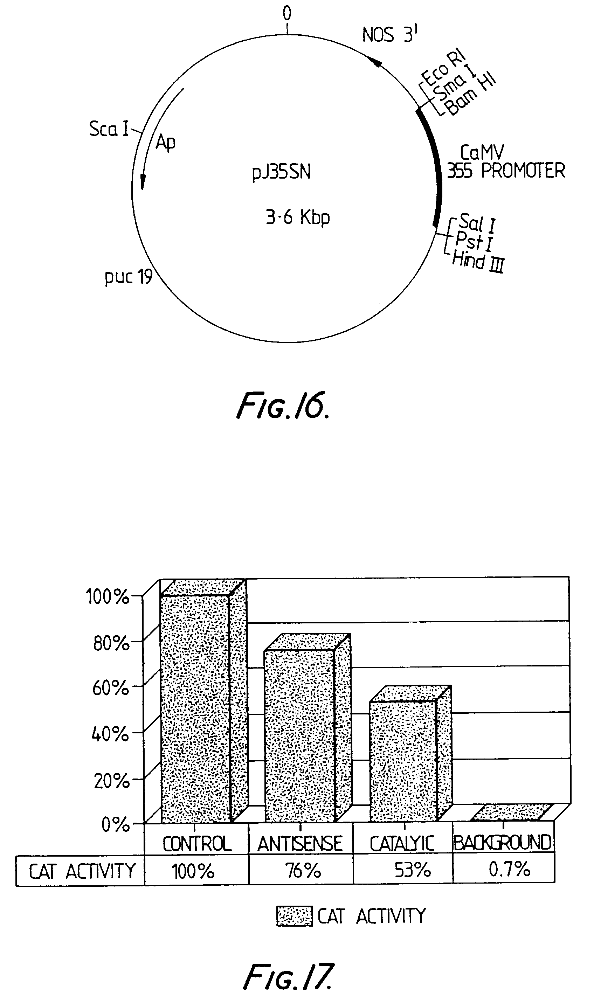

FIGURE 16 shows a map of plasmid pJ35SN; and

FIGURE 17 is a graphic presentation of the average of four experiments on the inhibition of CAT expression in plants (tobacco protoplasts).

The following Examples are given to illustrate the present invention and are not to be construed as limiting the present invention.

Reactions and manipulations involving DNA, such as ligations, restriction enzyme digestions, bacterial transformation, DNA sequencing etc. were carried out according to standard techniques, such as those described by Maniatis et al (Molecular Cloning, Cold Spring Harbour, 1982). Manipulations involving RNA were also carried out according to standard techniques, such as those described by Uhlenbeck (Nature 328, 596-600 (1987)) and Haseloff and Gerlach (Nature 334, 585-591 (1988)).

## EXAMPLE 1

### Self-Catalyzed Cleavage of Mutated sTobRV RNA:

A concensus of the domains associated with naturally-occurring RNA cleavage sites in ASBV, newt satellite DNA transcripts and the satellite RNAs of sTobRV, LTSV, velvet tobacco mottle virus (VMoV), solanum nodiflorum mottle virus (SNMV) and subterranean clover mottle virus (SCMoV) as shown in Figure 1a. Nucleotide sequences which are conserved between these structures are shown, while non-conserved sequences are represented as X. An extra U is positioned after residue [l]A in LTSV(+) strand.

The domain associated with the self-catalysed cleavage of the (+) strand of sTobRV was studied to ascertain the enzymic substrate activity within this domain. First, cloned sTobRV cDNAs were mutagenised using an oligonucleotide linker (BamHI) insertion protocol.

### Construction of a Vector for in vitro Expression of sTobRV:

A 160 bp Taq I - Spe I fragment of sTobRV cDNA was isolated from pSP653 (Gerlach et al. 1985, Virology 151; 172-185) and ligated to Acc I - Spe I cut, phosphatase-treated pGEM 4 to reform the Acc I site. A resulting clone was linearized with Acc I, phosphatase-treated and a 359 bp Taq I fragment of the sTobRV cDNA was inserted. The resulting clones were screened for the presence of a circularly permuted 520 bp sTobRV cDNA sequence containing the terminally redundant residues 277 to 81 (pTTS). The sTobRV sequence is flanked by promoters for T7 and SP6 RNA polymerases, and in vitro transcription gave rise to RNAs of (+) or (-) orientation which contained two sites for self-cleavage.

### In vitro Mutagenesis:

The plasmid pTTS (50 ug) was linearized with BamH I, treated with SI nuclease and religated, to remove a unique BamH I site. The resulting construction, pTTS-B, was treated with $2 \times 10^{-4}$ units DNase I in 20mM Tris-HCl pH 7.0, 15 mM $MnCl_2$ for 10 mins. at 37°C. The resulting linear DNAs were trimmed and/or end-filled using T4 DNA Polymerase, and purified by 0.7% LGT agarose gel electrophoresis and extraction. Kinased BamH I linker sequences (CGGATCCG) were ligated to the linearized plasmid overnight at room temperature in the presence of 5% polyethylene glycol. Subsequently, the reactions were BamH I digested, and the linear plasmid DNAs repurified by 0.7% LGT agarose gel electrophoresis (this was found necessary to remove last traces of circular plasmid, together with unligated linkers). Plasmids were recircularized using T4 DNA ligase and transformed into E. coli DH-I. Colonies (greater than 1000) were scraped from agar plates, grown in liquid culture to saturation and a mixed population of plasmid DNAs prepared. The mixed sTobRV cDNA inserts were excised by restriction enzyme digestion at flanking EcoRI and PstI sites, purified by 1% LGT agarose gel electrophoresis, and sub-cloned into EcoRI - PstI cut, phosphates-treated pGEM 4. The resulting transformants were again pooled, grown in liquid culture and plasmid DNA prepared. The plasmid DNAs were treated with BamHI, to cleave only those plasmids containing a BamHI linker sequence, and the linear forms were again purified by two rounds of 0.7% LGT agarose gel electrophoresis, recircularized with T4 DNA ligase, and transformed into E. coli DH-I. Individual transformants were screened for the approximate position of the inserted BamHI linker within the sTobRV sequence by restriction enzyme digestion, sub-cloned into M13 mp19 and sequenced via the dideoxynucleotide chain termination technique.

A library of sTobRV mutants resulted, and nucleotide sequence analysis showed that each mutant contained an inserted BamHI linker sequence (CGGATCCG) together with flanking duplicated or deleted sTobRV sequences. The mutants were transcribed in vitro and the RNAs assayed for their ability to undergo cleavage. From these experiments, a 52-nucleotide sequence was identified as containing both the substrate and cleavage portions of sTobRV RNA. This 52-nucleotide sequence, depicted in Figure 1b, contained the domain of conserved sequence required for self-cleavage of other RNAs (Figure 1a). One mutant, designated D-51, contained an eight nucleotide BamHI linker sequence inserted between three duplicated sTobRV nucleotides numbered 7 to 9. This mutant underwent self-catalysed RNA cleavage.

97 and 108 base-pair HaeIII fragments containing the 52-nucleotide cleavage sequence of the wild type and D-51 RNAs (as shown in Figures 1b and 1c) were excised from sequenced plasmid clones. The fragments were ligated into the SmaI site of pGEM4 and screened to obtain both orientations of the insert. The plasmids were linearised using EcoRI and (+) and (-) strand RNAs of lengths 159 and 170 bases were transcribed using 200 units/ml T7 RNA polymerase in 50 mM Tris-HCl, pH 7.5, 10 mM NaCl, 6 mM $MgCl_2$, 2 mM spermidine, 1000 units/ml RNasin, 500 $\mu$M ATP, CTP and GTP with 200 $\mu$M [$\alpha^{32}$P] UTP. RNAs were fractionated by electrophoresis on a 10% polyacrylamide, 7 molar urea, 25% formamide gel, and autoradiographed.

As shown in Figure 1d, no cleavage of the (-) strand RNA transcripts was observed. This was as expected, as the (-) strand did not contain a self-catalysed cleavage site. With the (+) strands of both the wild type and D-51 sequences, cleavage took place, with cleavage of the D-51 RNA being somewhat less efficient than that of the wild type (Figure 1d). This experiment indicates that the single stranded loop region at the right-hand side of the 52-nucleotide sequence involved in the self-catalysed cleavage of RNA is non essential.

## Separation of Enzymic and Substrate Activities:

Using the BamHI restriction endonuclease site inserted into D-51, the flanking HaeIII-BamHI and BamHI-HaeIII fragments were obtained and each was sub-cloned into an E. coli plasmid suitable for in-vitro transcription. This led to the elimination of the mutated single-stranded loop from the self-cleavage domain, splitting the region into two RNA segments (Figure 2a). The smaller HaeIII-BamHI fragment contained nucleotides 321 to 9, including the actual site of cleavage and was termed the S fragment. The BamHI-HaeIII fragment containing sTobRV nucleotides 7 to 48 was termed the ribozyme or Rz fragment. The E. Coli plasmids used for in-vitro tanscription were pGEM3 and pGEM4 (Promega, Madison, WI, U.S.A.). These expression plasmids contain:

(a) an origin of replication;
(b) selectable drug resistance (Amp$^r$) gene;
(c) a multiple cloning site flanked by RNA polymerase promoters which can be used for <u>in vitro</u> production of transcripts.

T7 DNA polymerase treated, KpnI digested Rz-pGEM3 and XbaI digested S-pGEM4 were transcribed using SP6 RNA polymerase under the same conditions as set out above.

As shown in Figure 2, both the S and Rz-RNAs showed no significant degradation when incubated alone (Figures 2b, lanes 1 and 3) under conditions suitable for highly efficient self-cleavage (50°C, 20mM $MgCl_2$, pH 8.0). The labelled Rz-RNA also appeared unaltered after incubation with the S-RNA (Figure 2b, lanes 2 and 5). However, when the S-RNA was mixed with the Rz- RNA, efficient cleavage of the S-RNA occurred (Figure 2b, lanes 4 and 5) producing two fragments. The product sizes were consistent with cleavage of the S-RNA (84 bases) at the normal site between nucleotides #359 and #1, to give 5' and 3' proximal fragments of 67 and 17 nucleotides, respectively. This shows that the S-RNA acted as a substrate for ribonucleolytic cleavage by the Rz-RNA, which acted in a catalytic fashion.

A model of a ribozyme based on the catalytic region of sTobRV RNA is shown in Figure 3. The ribozyme has two arms or flanking sequences of single stranded RNA shown at C, hybridizing to complementary sequences on a substrate RNA, i.e. RNA to be cleaved. Each flanking sequence shown at C, contains 8 ribonucleotides. The number of nucleotides contained in region C is not critical. Sufficient nucleotides must, however, be present to allow the ribozyme to hybridize to a target RNA. Four nucleotides in each region C appears to be the minimum number for hybridization.

The catalytic region B contains sequences which are highly conserved in naturally-occurring cleavage domains (see Figure 1a). From a comparison with cleavage domains of the known sequences, the length of the base pair stem II is unimportant, as is the presence of an associated loop at one end thereof.

The cleavage site within the target RNA is depicted at A (in Figure 3) as GUC. On the basis of our experiments (not shown), and others by Koizumi (FEBS LETT 288; 228-230 (1988); and FEBS LETT 239; 285-288 (1988)) the identified cleavage sites in naturally occurring RNAs, the sequences GUA, GUC, CUC, AUC and UUC also act as cleavage sites within RNA.

**EXAMPLE 2**

**Demonstration of the Design, Synthesis and Activity of Ribozymes with New and Highly Specific Endoribonuclease Activity:**

As an illustration of this invention, three ribozymes have been designed, which are targeted against the transcript of a commonly used indicator gene derived from bacteria, Tn9 Chloramphenicol Acetyl Transferase (CAT), which can provide antibiotic resistance in bacteria, plants and animals and can be easily assayed. These ribozymes, designated RzCAT-1 to 3 correspond to potential GUC cleavage sites in CAT RNA at positions 139-140, 494-495 and 662-663 respectively. The sequences of these ribozymes are depicted in Figure 4. In each case, the flanking sequences of the ribozyme which hybridize to the target CAT RNA, were 8 nucleotides in length. The catalytic region was chosen to correspond to that of sTobV RNA, shown in Figure 3.

The CAT gene was obtained from pCM4 and sub-cloned as a BamHI fragment into pGEM-32 (from Promega, Madison, WI, U.S.A.). This plasmid was linearised with HindIII and CAT gene transcripts were obtained using T7 RNA polymerase with 220uM [$\alpha$-$^{32}$P]UTP. Ribozyme sequences were synthesised as oligodeoxynucleotides, Rz CAT-1, 2 and 3, respectively. They were kinased, ligated with phosphatased treated, EcoRI-PstI cut pGEM4 and incubated with the Klenow fragment of DNA polymerase I before bacterial transormation. EcoRI linearised plasmids were transcribed with T7 RNA polymerase to produce ribozyme RNAs. Ribozymes were incubated with CAT transcript in 50 mM Tris-HCl pH 8.0, 20 mM MgCl$_2$ at 50°C for 60 min, and the products fractionated by 5% polyacrylamide 7M urea, 25% formamide gel electrophoresis prior to autoradiography.

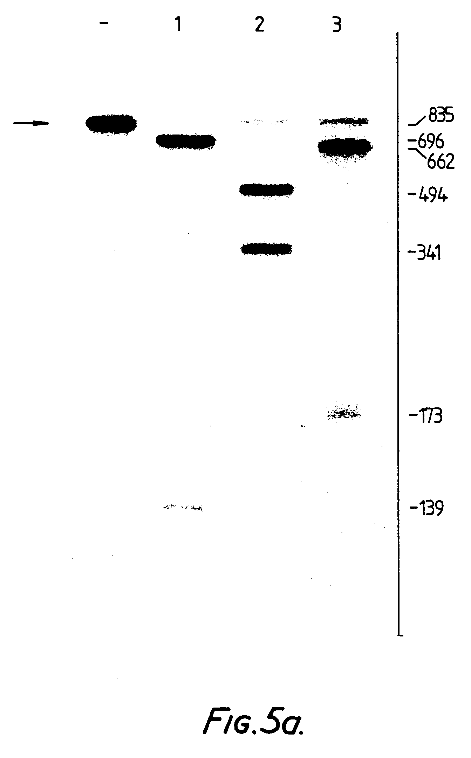

When the 840-nucleotide CAT transcript was incubated with any one of the three ribozymes, efficient and highly sequence-specific cleavage occurred (Figure 5) producing 2 RNA fragments in each reaction. The fragment sizes were consistent with the predicted sites for cleavage (i.e. 139 and 696, 494 and 341, 662 and 173 base fragments were the 5' and 3' products from RzCAT-1 to 3 catalysed cleavage respectively). The conditions required for these ribozyme-catalysed cleavages were similar to those observed for naturally occuring cleavage reactions (Foster, A.C. and Symons, R.H., Cell 49: 211-220 (1987) and Foster, A.C. and Symons, R.H. Cell 50: 9-16 (1987)), with more efficient cleavage occuring at elevated pH, temperature and divalent cation concentrations (data not shown). When present in molar excess, the three ribozymes catalysed almost complete cleavage of the CAT RNA substrate after 60 min. in 50 mM Tris HCl, pH 8.0, 20 mM MgCl$_2$ at 50°C. Under similar conditions with 0.1 $\mu$M substrate and 3 $\mu$M ribozymes, the $T_{1/2}$ of CAT mRNA substrate was 3.5, 3.5 and 2.5 min. in the presence of RzCAT-1 to 3 respectively. The ribozyme sequences were inactive against the complement of the substrate RNA (i.e. the (+) strand), and in the form of oligodeoxyribonucleotides (data not shown). The 3' terminal cleavage fragments from each ribozyme catalysed reaction were isolated and 5' $^{32}$P-kinased (50 mM TrisHCl pH 9, 10 mM MgCl$_2$, 10 mM DTT with 50 uCi $\gamma$-$^{32}$P ATP and 5 units T4 polynucleotide kinase for 30 min. at 37°C.). Efficient kinasing of the fragments indicated that they possessed 5' terminal hydroxy groups, similar to those produced in naturally occuring cleavage reactions.

The terminal nucleotide of the fragments produced by cleavage of the CAT sequences by RzCAT-1 to 3 were determined. Briefly, radiolabelled fragments were purified on a 5% polyacrylamide gel and digested with an equal volume of 500 units/ml RNase T1, 25 units/ml RNase T2 and 0.125 mg/ml RNaseA in 50 mM ammonium acetate pH 4.5 for 120 min. at 37°C. The products were fractionated on a 20% polyacrylamide gel containing 25 mM sodium citrate, pH 3.5 and 7 molar urea. Figure 5b shows that the cleavage of the CAT sequences by RzCAT-1 to 3 occurs precisely before nucleotides A, U and G respectively.

The terminal sequence of the CAT gene fragments were determined directly using the partial enzymatic digestion technique (Donis-Keller et al., Nucleic Acids Res. 4: 2527-2538 (1980)), using base-specific partial ribonucleolytic cleavage. The sequence of the fragments confirmed that cleavage occurred at the expected locations within CAT RNA (not shown).

**Enzymatic Catalysis:**

To demonstrate that ribozymes cause cleavage of the CAT mRNA substrate in a catalytic manner, each was incubated with a molar excess of substrate, under conditions which should favour both efficient cleavage and product dissociation. Figure 6 shows the results of an experiment where after 75 min. at 50°C, pH 8.0 in 20 mM MgCl$_2$, 10 pmoles of RzCAT-1 had catalysed specific cleavage of 163 pmoles of a truncated CAT mRNA (173 bases) substrate to give 5' and 3' fragments of 139 and 34 bases, respectively. On average, each ribozyme had participated in greater than ten cleavage events. After 75 minutes at 50°C some non-specific cleavage of RNA was noticed due to the extreme conditions, but 70% of the remaining intact RNAs (163 pmoles) had accumulated as the 139 base fragment. Similar results were obtained for RzCAT-2 and 3 (data not shown), and thus each acts as an RNA enzyme.

## EXAMPLE 3

### The Effect of Temperature on Ribozyme Activity:

The effect of reaction temperature on the in-vitro rate of ribozyme activity was examined.

A time course of reactions for ribozymes RzCAT-1 to 3 on CAT RNA substrate at 37°C and 50°C was carried out.

In this experiment, reactions for each ribozyme were set up in duplicate, using reaction conditions for ribozyme cleavage set out in Example 2. One reaction was incubated at 37°C, the other at 50°C. Samples were removed at time points to 90 minutes and the extent of reaction was analysed by denaturing polyacrylamide gel electrophoresis. Figure 7 shows the time course of reaction for each of the ribozymes RzCAT-1 to 3 at 37°C and 50°C. The reaction rate of each ribozyme increases with increased reaction temperature.

The time taken for 50% ($t_{1/2}$) cleavage of CAT RNA is set out in Table 1.

TABLE 1

|  | RzCAT-1 | RzCAT-2 | RzCAT-3 t1/2 (mins.) |
|---|---|---|---|
| 50°C | 3.5 | 3.5 | 2.5 |
| 37°C | 55.0 | 70.0 | 65.0 |

As shown in Table 1, the rate of reaction of ribozymes at 37°C is approximately 20 times slower than the rate of reaction at 50°C.

## EXAMPLE 4

### The Effects of Varying Arm Lengths of Ribozymes (or Flanking Sequence) on Ribozyme Catalytic Activity:

The arms or flanking sequences of a ribozyme (region (I) of formula 1) hybridize the ribozyme to a target RNA whereafter cleavage of the RNA takes place. In this experiment, the effect on cleavage rate of a target sequence by altering the extent of complementarity and subsequent length of base pairing of the ribozyme arms to the target sequence was investigated.

Ribozymes were produced with 4, 8 and 12 base complementarity to the target sequence RzCAT-2 on each arm (Figure 8a). The ribozymes were prepared according to the methods of Example 2. Ribozyme activity was determined by incubating the ribozyme RNA with CAT RNA as described previously.

The ribozyme having a 4 base complementarity on each arm did not cleave the substrate RNA. The ribozyme with 8 base complementarity on each arm cleaved the CAT substrate as did the ribozyme having 12 base complementarity. Ribozymes with 12 base complementarity cleaved target RNA more efficiently, as judged by reaction rate in-vitro than did ribozymes having a lesser number of base complementarities. Even though it appears necessary to have more than four base complementarity, increasing the length of the hybridizing region of ribozymes increases their reaction rate.

In a second experiment, the reaction efficiency of a ribozyme having (a) complementarity to the entire length of the CAT transcript target RNA and (b) multiple catalytic domains, was investigated.

Four GUC target sites in CAT RNA sequences were chosen. Ribozyme catalytic domains against these sites were "inserted" into a complete anti-sense (-) sequence for the CAT transcript and catalytic activity tested.

The four sites chosen were the three specified by RzCAT-1 to 3 described previously, and a further site which may be represented as follows:

```
                   New CAT site
                   #192
                   His   His   Ala   Val   Cys   Asp   Gly
              5'   CAU   CAU   GCC   GUC   UGU   GAU   GGC   3
```

where "192" refers to amino acid 192 in the CAT polypeptide and an refers to the site of cleavage.

Oligodeoxyribonucleotides containing ribozyme catalytic domains and spanning each of these cleavage sites were used for M13 mutagenesis experiments to produce a sequence containing the entire complement of the CAT sequence but with the four ribozyme catalytic domains inserted within it. M13 mutagenesis was performed by binding of oligonculeotides containing ribozyme insertions to single stranded M13 DNAs containing uracil, followed by synthesis of complementary DNAs containing the insertion. The complementary DNAs were recovered following cloning in an appropriate Escherichia coli strain, (T.A. Kunkel 1985, Proc. Natl. Acad. Sci. U.S.A. 82: 488-492). The resultant double-stranded cDNA was cloned in an in-vitro expression vector to produce ribozyme RNA using the T7 polymerase transcription system. Ribozyme activity was determined by incubation of ribozyme RNA with CAT transcript followed by gel electrophoresis of the reaction mixture, after glyoxal treatment to denature nucleic acids.

Autolytic cleavage occurred at all the expected sites on the CAT transcript. Accordingly, the flanking sequences of arms or a ribozyme may extend along the full length of the RNA transcript which is to be cleaved.

Figure 9 schematically shows the production of catalytic anti-sense RNA containing each of the four ribozymes. The catalytic anti-sense RNA contains approximately 900 bases.

Under the above reaction conditions, the ribozyme and target sequences form high molecular weight complexes presumably by extensive base pairing. A strong denaturing treatment such as glyoxal treatment is required to resolve reaction products during electrophoresis.

**EXAMPLE 5**

**Target Sequences for Ribozyme Cleavage:**

The GUA motif in mRNA was tested to see whether a ribozyme would effect RNA cleavage at this sequence.

A specific site in CAT mRNA, including the GUA motif (Figure 10a) was chosen and an appropriate ribozyme sequence was prepared and tested for activity. The ribozyme contained arms of 8 ribonucleotides.

Synthetic oligonucleotides corresponding to the ribozyme of Figure 10 were prepared according to Example 2 and double-stranded cDNA cloned into an in-vitro expression vector (pGEM 4, see above) in E. coli in order to produce ribozyme RNA using the T7 polymerase transcription system. Ribozyme activity was determined by incubation of ribozyme RNA with CAT mRNA, followed by gel electrophoresis of the reaction mixture as previously described.

The ribozyme effected cleavage at the GUA target site (not shown). Accordingly, the motif GUA in RNA is a substrate for ribozymes of the present invention. This was not completely unexpected since one naturally occuring cleavage site in the satellite RNA of lucerne transient streak virus requires recognition of a GUA site.

Similarly, a GUU motif in the CAT RNA target sequence was tested with an appropriate ribozyme (See Figure 10b), and cleavage was effected.

**EXAMPLE 6**

**Ribozyme Cleavage of Viral RNA:**

Viroid RNA, in the form of citrus exocortis viroid RNA, was cleaved using a ribozyme of the present invention.

Two GUC target sites were chosen in citrus exocortis viroid (CEV) RNA. One site in the complementary strand sequence was also chosen. Ribozymes were prepared against all of these sites and tested for activity. The ribozymes were designated CEV9x(+), CEV9x(-) and CEV25x(+). Figure 11 shows the three cleavage sites in CEV RNA for each of these ribozymes.

Ribozymes were prepared according to previous methods. Ribozyme RzCEV25x(+) is shown in Figure 12. This ribozyme cleaves the GUC motif at nucleotide 116 of CEV RNA.

Figure 12(b) shows cleavage of CEV RNA with ribozyme RzCEV9x(+). No cleavage is observed with ribozyme RzCEV9x(-).

This experiment indicates that ribozymes are active against target RNA sequences from diverse sources. This is to be expected, as all RNAs are formed from the basic ribonucleotide building blocks of adenine, guanine, cytosine and uracil, regardless of their animal, plant or microbial origin.

## EXAMPLE 7

### Examples of Ribozymes Having Variable Catalytic Domains

A ribozyme targeted against a CAT-2 site was prepared using the catalytic domain sequence from the satellite RNA of subterranean clover mottle virus (SCMoV). Twelve base complementarity of ribozyme arm flanking sequence was incorporated into the design of the ribozyme RzSCMoV. The ribozymes RzCAT-2 and RzSCMoV are shown at Figures 13a and 13b respectively. The loop region of RzSCMoV contains 5 nucleotides, having the sequence AAAUC. This is to be contrasted with the loop region of RzCAT-2 which contains 4 nucleotides having the sequence AGAG. In addition, RzSCMoV contains a C in the catalytic region, in place of U* in RzCAT-2. The different sequences in RzSCMoV when compared with RzCAT-2 are marked.

The RzSCMoV was produced according to Example 2. RzSCMoV was active, yielding two cleavage products as expected.

In another experiment, the citrus exocortis viroid (CEV) target site at nucleotide -336 in its complementary RNA was cleaved using a ribozyme (RzCEV2) having the sequence set out in Figure 14a. The loop region designated with the letter "L" in Figure 14 comprises six nucleotides having the sequence 3'-CCTATA-5'. This is distinct from the loop region of sTobRV which comprises four nucleotides having the sequence 3'-AGAG-5'. This ribozyme cleaves target CEV complementary RNA at position -336 as shown in the electrophoretic profile of Figure 14b.

This experiment indicates that the number of nucleotides, and nucleotide sequence of the loop region is unimportant in ribozyme activity. In these experiments, the ribozyme was produced according to methods previously described in the specification.

In another experiment, the effect of base pairing in the catalytic domain (stem region) on ribozyme activity was investigated.

A modified ribozyme corresponding to RzCAT-2 but containing four extra base pairs was prepared and tested. In Figure 15a, the sequence of the ribozyme Rz CAT-2 is shown hybridized to target CAT RNA. The test ribozyme is shown at 15b, with the additional base pairs boxed. The test ribozyme had comparable activity to that of RzCAT-2. This indicates that the base paired region of the ribozyme catalytic domain may be of variant length, without effecting catalytic activity.

We have observed (data not shown) that the stable in vivo form of sTobRV RNA transcripts expressed in transgenic plants is primarily circular, presumably due to ligation of 5' and 3' termini. Therefore, the use of two autolytic cleavage sites flanking a sequence of interest in an in vivo RNA transcript is likely to lead to a circularized product which may have greater stability than linear transcripts. This approach appears to provide a novel method for in vivo stabilization of ribozyme sequences. This is termed circularization.

## EXAMPLE 8

### In-vivo Activity of Ribozymes:

The in-vivo activity of ribozymes in plant cells is investigated in this Example.

### Experimental Protocol:

Plasmids containing anti-CAT (CAT = chloramphenicol acetyl transferase) or combined anti-CAT/ribozyme gene constructions (see below) were introduced into tobacco protoplasts in the same amount and proportion relative to each other, along with another plasmid which contained a functional CAT gene construction. CAT activities were measured and compared with the base level of gene activity.

## Materials and Methods:

(a) Electroporations and CAT assays.

These were performed as described in Llewellyn et al. J. Mol. Biol. (1987) 195:115-123. Briefly, protoplasts of <u>Nicotiana plumbaginifolia</u> line T5 were prepared from a suspension two days after subculture, suspended in 10mM HEPES, pH 7.2, 150mM NaCl, 0.2M mannitol and adjusted to a density of 3 x 10⁶/ml. Electroporation was carried out using a single 50ms pulse at 250V. Protoplasts were diluted 10-fold and cultured and for 20 hr. at 26°C in the dark. They were disrupted by sonication and extracts obtained. The extracts were normalized for protein content and assayed for CAT activity <u>in vitro</u> using $^{14}$C-chloramphenicol and acetyl CoA. Reaction products were separated by thin layer chromatography and visualized by autoradiography. Extent of reactions were calculated by the production of radioactive product derivatives from the $^{14}$C-chloramphenicol template.

(b) Gene Constructions.

Gene constructions were introduced into 0.1 ml protoplast suspensions as plasmid DNAs which had been purified from bacteria by extraction and two cycles of CsCl equilibrium density gradient centrifugation. They were resuspended in 10 mM Tris/1 mM EDTA/pH = 7.5 for use.

The active CAT gene construction was borne on the plasmid designated pCAT7 +. It was derived by fusion of a CAT gene sequence (from plasmid pCM4, see T.J. Close and R. Rodriguez, 1982, Gene 20:305-316) into the plasmid pJ35SN (derived from p35SN, W.L. Gerlach et al., 1987, Nature 328:802-805) so that the active gene construction was:

35S refers to the 35S CaMV (cauliflower mosiac virus) promoter, NOS to nopaline synthetase polyadenylation signal, T/C to transcription.

Along with 0.2 ug of pCAT7 + there were added various gene constructions in excess as described below. The gene constructions were contained within plasmids with the following designations:

**pJ35SN -** This vector plasmid, a map of which is shown in Figure 16, contains a 35S CaMV promoter and plant nopaline synthase 3' polyadenylation signal, which may be depicted as follows:

**pCAT7- -** This contains the CAT gene sequence inserted into pJ35SN such that transcription will result in the production of the antisense CAT RNA, which may be depicted as follows:

**pCAT19- -** This contains the CAT gene with four catalytic ribozyme domains included within it, (see Example 4 and Figure 9), inserted into pJ35SN such that transcription will result in the production of antisense CAT RNA containing four catalytic ribozyme domains, which may be depicted as follows:

13

```
5'                                                    3'
  ┌──────┬──────────────←──────────┬──────┐
  │ 35S  │            CAT          │ NOS  │
  └──────┴─────────────────────────┴──────┘
        catalytic domain insertions
```

**Results:**

The following table shows the relative CAT activities in cells 20 hours after electroporation. Activity is expressed as per cent conversion of chloramphenicol substrate in a 1 hour assay.

| Treatment | ug Plasmid Electroporated | | | | % Conversion |
|---|---|---|---|---|---|
| | pCAT7+ | pJ35SN | pCAT7- | pCAT19- | |
| 1A | | | | | 0 |
| 1B | | | | | 0 |
| 2A | 0.2 | 18 | | | 21 |
| 2B | 0.2 | 18 | | | 46 |
| 3A | 0.2 | 9 | 9 | | 28 |
| 3B | 0.2 | 9 | 9 | | 32 |
| 4A | 0.2 | | 18 | | 26 |
| 4B | 0.2 | | 18 | | 19 |
| 5A | 0.2 | 9 | | 9 | 19 |
| 5B | 0.2 | | | 9 | 22 |
| 6A | 0.2 | | | 18 | 14 |
| 6B | 0.2 | | | 18 | 16 |
| (for each treatment "A" and "B" are duplicates) | | | | | |

The following conclusion can be drawn from these results:

(a) The introduction of the CAT gene construction results in significant CAT activity - compare 2A,B with 1A,B. There is variability between duplicates. From the trends seen in the other samples (see "b" and "c" below) it is likely that 2A shows an abnormally low activity.

(b) Concomitant introduction of an antisense gene construction results in a decrease in the level of activity - compare 3A,B and 4A,B with 2B. The extent of the decrease is related directly to the level of the antisense gene added as plasmid - compare 3A,B and 4A,B.

(c) Concomitant introduction of the combined antisense/ribozyme gene construction results in a decrease in gene activity - compare 5A,B and 6A,B with 2B. Furthermore, the decrease is more marked than for the corresponding levels of antisense gene constructions - compare 5A,B with 3A,B, and 6A,B with 4A,B.

The average results for four in-vivo experiments are shown in Figure 17. In this Figure, "control" represents treatment 2. "Antisense" represents treatment 4. "Catalytic" represents treatment 6 and "Background" represents treatment 1.

The catalytic ribozyme inhibits CAT activity an average of 47%, compared to an average of 34% for an antisense ribozyme.

The introduction of ribozyme-bearing genes into plant cells inhibits the activity of genes against which they are targeted. Furthermore, the inhibition is greater than for corresponding antisense RNA molecules.

These results show that ribozymes will be active in animal, plant or microbial cells against a range of target RNA molecules.

The mechanisms of action of the ribozymes in this Example is unclear. For example, the antisense ribozyme may irreversibly hybridize to a target RNA and catalyse phosphodiester bond cleave at one or more selected target sites along the target RNA. Alternatively, cellular enzymes may unwind the antisense RNA from its target sequence, such that the target RNA is cleaved into two or more fragments.

**EXAMPLE 9**

**In-vivo Activity of Ribozymes in Animal Cells:**

The activity of ribozymes in inactivating a target RNA in mammalian cells is demonstrated in this Example.

**Materials and Methods:**

The active gene constructions encoding ribozymes were transfected into the widely available monkey kidney cell line COSI by electroporation. In this method, $3 \times 10^6$/ml COSI cells suspended in a buffered saline with 10% FCS (foetal calf serum), were contacted with various gene constructs and an electric discharge applied to effect electroporation of DNA into the cells. The transfected cells were incubated at 37°C for 48 hours in culture medium before assay for CAT and luciferase activity.

CAT gene constructs were borne on the plasmid designated pTK CAT (Miksicek et al., Cell 46: 283-290, 1986). This plasmid was derived by the introduction of a CAT gene sequence into the plasmid pSV2 such that it is under the control of the thymidine kinase promoter of the herpes simplex virus.

Gene constructs encoding ribozymes were borne on the plasmid pSV232A (De Wet et al., Molecular and Cellular Biology 7: 725-737, 1987) containing the luciferase gene fused to the SV40 early promoter. DNA encoding ribozymes was ligated into the XbaI site at the 3' end of the luciferase gene according to the standard methods of Maniatis et al. (Molecular Cloning, A Laboratory Manual, Cold Spring Harbour, 1982).

The following constructs were prepared using standard techniques of Maniatis et al. (Supra):

**pFC58 -**  This plasmid vector contains DNA encoding ribozyme RzCAT-1 fused to the 3' end of the luciferase gene in a non-functional orientation.

This may be depicted as follows:

| 232A | SV40 early | ▶ | luciferase | ▶ | ◀ ribozyme RzCAT-1 | small T | Poly$^+$ | 232A |

where 232A refers to pSV232A sequences, SV40 early refers to the early promoter of SV40 and small T is DNA encoding the small T intervening sequence of SV40. This construct results in production of an RNA molecule encoding luciferase and the ribozyme RzCAT-1, the latter being in an orientation such that it would not be expected to be catalytic.

**pFC4 -**  This plasmid is the same as pFC58 except that RzCAT-1 is replaced with RzCAT-3.

**pFC1-6 -**  This plasmid is the same as pFC58 except that RzCAT-1 is replaced with RzCAT-3 in the sense orientation (5'-3').

**pFC20 -**  This plasmid is the same as pFC1-6 except that RzCAT-3 is replaced with RzCAT-2 having eight nucleotide flanking sequences.

**pFC12 -**  This plasmid is the same as pFC20 except that the ribozyme RzCAT-2 contains twelve nucleotide flanking sequences.

**pFC50 -**  This plasmid contains the CAT gene with four catalytic ribozyme domains included within it (see Example 4 and Figure 9) in the sense orientation (5'-3'), which on transcription gives rise to an inactive ribozyme. This plasmid may be depicted as follows:

| SV40 late | CAT gene containing ribozyme domains → | Poly A+ |

non-catalytic RNA

**pFC54 -**  This plasmid is the same as pFC50, except that the CAT gene and the ribozyme domains are in the antisense (3'-5') active orientation.

**pFC64 -**  This plasmid shares the SV40 promoter and polyadenylation signals with pFC50 and contains the wild type CAT gene with no inserted ribozyme domains. This gene is in an antisense orientation, and thus does not produce CAT protein.

**pFC65 -** This plasmid is the same as pFC64 except that the wild type CAT gene is in the sense (5'-3') orientation and is thus productive of CAT protein.

Assays:

Luciferase activity was assayed according to the methods of De Wet et al. (Supra). Briefly, COS cells were lysed 48 hours after transfection, and the cell lysate incubated with luciferin, the substrate of luciferase, and luminesence detected using a scintillation counter.

CAT activity was also measured using COS cell lysates (cell lysates were divided into two, and each portion assayed either for luciferase or CAT activity), according to the method of Sleigh, M.J., Anal. Biochem. 156: 251-256, (1986).

In the in-vivo assays, pFC58 and pFC4 did not affect CAT activity in transfected cells. This activity was designated 100% CAT activity and 0% CAT suppression. CAT activity in cells transfected with other plasmids was measured relative to pFC58. The % of CAT suppression was measured as

$$100 \quad - \quad \left( \frac{CAT_{test}}{CAT_{control}} \right) \quad x \quad 100$$

normalised to luciferase production. $CAT_{test}$ = CAT assay result for test constructs. CAT control = CAT assay for control constructs (pFC4 and pFC58).

Luciferase production is an internal control for electroporation, and gives a measure of ribozyme production within each individually electroporated tissue culture plate.

Results:

Experiment (i)

| Treatment | μg plasmid electroporated/1.5x10⁶ cells | | | | | % CAT Suppression |
|---|---|---|---|---|---|---|
| | pTKCAT | pFC58 | pFC20 | pFC1-6 | pFC12 | |
| 1 | 5 | | 2 | | | 56 |
| 2 | 5 | | 1 | 1 | | 53 |
| 3 | 5 | | | | 2 | 40 |
| 4 | 5 | 2 | | | | 0 |

All treatments were carried out in duplicate and an average value given.

Experiment (ii)

| Treatment | μg plasmid electroporated/1.5x10⁶ cells | | | | | % CAT Suppression |
|---|---|---|---|---|---|---|
| | pTKCAT | pFC-1-6 | pFC20 | pFC12 | pFC4 | |
| 5 | 5 | 2 | | | | 75 |
| 6 | 5 | | 2 | | | 75 |
| 7 | 5 | | 4 | | | 62 |
| 8 | 5 | 1 | 1 | | | 70 |
| 9 | 5 | | | 4 | | 51 |
| 10 | 5 | | | | 2 | 0 |

Treatments 5 to 10 were carried out in duplicate and an average value given.

Experiment (iii)

| Treatment | $\mu$g plasmid electroporated/1.5x10$^6$ cells | | | % CAT Suppression |
|---|---|---|---|---|
| | pTKCAT | pFC1-6 | pFC4 | |
| 11 | 5 | 2 | | 66 |
| 12 | 5 | | 2 | 0 |

Treatments were carried out in quintuplicate and an average value given.

Experiment (iv)

| Treatment | $\mu$g plasmid electrophorated | | | | | % CAT Suppression |
|---|---|---|---|---|---|---|
| | pTKCAT | pFC50 | pFC54 | pFC64 | pFC65 | |
| 13 | 5 | 2 | | | | 0 |
| 14 | 5 | | 2 | | | 26 |
| 15 | 5 | | | 2 | | 2 |
| 16 | 0 | | | | 2 | NA |

Each of treatments 13 to 16 were carried out in quadruplicate.

The sense CAT construction (treatment 16) was productive of high levels of CAT activity in its own right. Therefore % suppression is not applicant (NA).

A number of experiments were also conducted with the TK promoter of pTKCAT replaced with the human metallothionein promoter. When this CAT construct was co-transfected into COSI cells with plasmids encoding one or more ribozymes, a marked decrease in CAT activity was observed.

The above results clearly demonstrate the in-vivo inactivating activity of ribozymes in animal cells.

Whilst the effectiveness of the ribozymes in vivo, is believed to be caused by one or more catalytic regions which are capable of cleavage of a target RNA, the presence of such regions in "Antisense" RNA type ribozymes may not actually lead to cleavage in vivo if the entire RNA/antisense RNA molecule does not fall apart. However, regardless of whether the molecule falls apart or not, the foregoing Examples demonstrate the effectiveness of the ribozyme in inactivating the target RNA. Thus, the invention is applicable to all ribozymes having a catalytic region capable of causing cleavage and a hybridizing region, regardless as to whether cleavage actually occurs in the target RNA. i.e. The hybridizing region may be so large as to cause the combined RNA/ribozyme to stay together and prevent the target RNA being cleaved into separated components even though the catalytic region is itself capable of causing cleavage.

Also in the present invention is disclosed (and was originally claimed in the parent application):

(a) A ribozyme comprising a hybridizing region which is complementary in nucleotide sequence to at least part of a target RNA, and a catalytic region which is adapted to cleave said target RNA wherein the hybridizing region contains 9 or more nucleotides.

(b) A ribozyme comprising one or more arms formed of single stranded RNA and having a sequence complementary to at least part of a target RNA, said one or more arms being associated with a catalytic region, capable of cleaving said target RNA; and where the hybridizing region comprises a single arm of RNA said arm contains at least 9 nucleotides, and where the hybridizing region comprises 2 or more arms of RNA, the sum of nucleotides in said arms is greater than 9 nucleotides.

(c) A ribozyme as defined in (b), having two single stranded arms of RNA, associated with one another through said catalytic region.

(d) A ribozyme as defined in any of (a) to (c) which does not modify cleaved target RNA.

(e) A ribozyme of the formula:

```
          3'                      5'
          (X)n X          X (X)n'
               A          C
               A          U
               G      lA  G
               C  *  G G  A
               |    |  X
               X'   X"
             5'        3'
```

wherein;

X represents any ribonucleotide and each X residue may be the same or different;

the sum of n and n' is greater than 6 and n and n' may be the same or different;

an (*) represents a base pair between complementary ribonucleotides;

X' and X" represent ribonucleotides of complementary sequence along at least part of their length to allow base pairing between the oligoribonucleotides, or X' and X" together form a single RNA sequence wherein at least part of said sequence comprises a stem formed by base pairing between complementary nucleotides;

and optionally, an additional nucleotide selected from any one of A, G, C or U is inserted after $^{l}$A.

(f) A ribozyme as defined in any of (a) to (e) having the formula:

```
          3'                      5'
          (X)n A          X(X)n'
               A          C
               A          U
               G      lA  G
               C  *  G G  A          (2)
               X  *  X X
               X  *  X
             (X)m *  (X)m'
               X      X
                  B
```

wherein;

X represents any ribonucleotide and each X residue may be the same or different;

an (*) represents a base pair between complementary ribonucleotides;

n and n' are as previously defined;

B represents a bond, a base pair, a ribonucleotide, or an oligoribonucleotide containing at least 2 ribonucleotides;

m and m' are 1 or more and may be the same or different; and

optionally, an additional nucleotide selected from any one of A, G, C or U is inserted after $^{l}$A.

(g) A ribozyme as defined in (e) or (f) where the sum of n and n' is 16 or greater.

(h) A ribozyme as defined in any of (a) to (g) wherein the catalytic region is capable of cleaving a target RNA after the sequence $X^{o}UY$ where $X^{o}$ is any ribonucleotide, U is uracil and Y is adenine, cytosine or uracil.

18

(i) A ribozyme containing multiple catalytic domains connected through flanking sequences, each said flanking sequence including sequences complementary to the target RNA which is desired to be inactivated.

(k) A ribozyme as defined in any of (a) to (i) which has been stabilized by circularization.

(l) A ribozyme comprised of catalytic antisense sequence as defined in any of (a) to (k).

(m) A method for the inactivation or cleavage of a target RNA which comprises reacting said target RNA with a ribozyme as defined in any of (a) to (l).

(n) A method as defined in (m), wherein said inactivation occurs in-vivo in prokaryotic or eukaryotic cells, as a result of transcription of said ribozyme from a DNA or RNA transfer vector transfected into said cells or precursors thereof, or expression of a gene encoding said ribozyme which is integrated into the host genome, such that said ribozyme hybridizes to and inactivates a desired cellular target RNA.

(o) A method as defined in (n), wherein said cells are plant or animal cells.

(p) A method as defined in (n) or (o),
wherein the target RNA is endogenous to the cells, or is exogenous, such as viral RNA.

(q) A method as defined in (p), wherein
target viral RNA is selected from RNA of the HIV(AIDS), hepatitis or other virus causing disease in mammals.

(r) A method as defined in (p), wherein
viral RNA is bacteriophage RNA.

(s) Use of a ribozyme according to any of (a) to (l) in the manufacture of a medicament for the treatment of viral disease in man or animals, said ribozyme being capable of hybridising to and inactivating transcripts of said viral RNA.

(t) A method for treating viral or viroid disease in plants which comprises administering to a plant a ribozyme according to any one of claims 1 to 11, said ribozyme hybridizing to and inactivating transcripts of said viral RNA.

(u) A composition which comprises a ribozyme as defined in any of (a) to (l) in association with pharmaceutically, veterinarially, or agriculturally acceptable carrier or excipient.

(v) A method for producing a ribozyme as defined in any of (a) to (l) which comprises the steps of: steps of:

    (a) ligating into a transfer vector comprised of DNA, RNA or a combination thereof a nucleotide sequence corresponding to said ribozyme;
    (b) transcribing the nucleotide sequence of step (a) with RNA polymerase; and
    (c) optionally purifying the ribozyme.

(w) A transfer vector comprised of RNA or DNA or a combination thereof containing a nucleotide sequence which on transcription gives rise to a ribozyme as defined in any of (a) to (l).

(x) A transfer vector as defined in (w), which is a bacterial plasmid or phage DNA.

(y) A prokaryotic or eukaryotic cell containing nucleotide sequences which on transcription thereof give rise to a ribozyme as defined in any one of (a) to (l).

## Claims

**1.** A compound having the formula:

wherein each X represents a nucleotide which may be the same or different;

wherein each of $(X)_n$ and $(X)_{n'}$ represents an oligonucleotide (a) capable of hybridising with an RNA target sequence to be cleaved and (b) defined by a predetermined sequence which sequence does not naturally occur covalently bound to the sequences X-A-A-G- and X-C-U-G-A-, respectively, such RNA target sequence not being present within the compound;

wherein each of n and n' represents an integer which defines the number of nucleotides in the oligonucleotide with the proviso that the sum of n + n' is sufficient to allow the compound to stably interact with the RNA target sequence through base-pairing;

wherein each * represents base pairing between the nucleotides located on either side thereof;

wherein each solid line represents a chemical linkage providing covalent bonds between the nucleotides located on either side thereof;

wherein a represents an integer which defines a number of nucleotides with the proviso that a may be 0 or 1 and if 0, the A located 5' of $(X)_a$ is bonded to the G located 3' of $(X)_a$;

wherein each of m and m' represents an integer which is greater than 1;

wherein each of the dashed lines independently represents either a chemical linkage providing covalent bonds between the nucleotides located on either side thereof or the absence of any such chemical linkage; and

wherein $(X)_b$ represents an oligonucleotide which may be present or absent with the proviso that b represents an integer which is greater than or equal to 4 if $(X)_b$ ispresent.

**2.** A compound of claim 1, wherein each X is a ribonucleotide.

**3.** A compound of claim 1, wherein the sum of n + n' is greater than or equal to 14.

**4.** A compound of any of claims 1 to 3, wherein each of n and n' is greater than 6.

**5.** A compound having the formula:

$$3'—[-(Y)_r—Q—(Y)_s-]_z—5'$$

wherein Q represents a compound of any of claims 1, 2, 3, or 4;

wherein each Y represents a nucleotide which may be the same or different;

wherein each of r and s represents an integer which may be greater than or equal to 0; and

wherein z represents an integer greater than or equal to 1.

**6.** The compound of any of claims 1, 2, 3, 4, or 5, wherein the RNA target sequence to be cleaved is a viral RNA sequence.

**7.** A composition which comprises the compound of any of claims 1, 2, 3, 4, 5, or 6, in association with a pharmaceutically, veterinarially, or agriculturally acceptable carrier.

**8.** A method for producing the compound of any of claims 1, 2, 3, 4, 5, or 6, which comprises the steps of:

(a) ligating into a transfer vector comprised of DNA, RNA or a combination thereof a nucleotide sequence corresponding to said compound;

(b) transcribing the nucleotide sequence of step (a) with an RNA polymerase; and

(c) recovering the compound.

**9.** A transfer vector comprised of RNA or DNA or a combination thereof containing a nucleotide sequence which on transcription gives rise to the compound of any of claims 1, 2, 3, 4, 5, or 6.

**10.** A composition which comprises the transfer vector of claim 9 in association with a pharmaceutically, veterinarially or agriculturally acceptable carrier.

**11.** A prokaryotic or eukaryotic cell comprising a nucleotide sequence which is, or on transcription gives rise to, the compound of any of claims 1, 2, 3, 4, 5, or 6.

**12.** A eukaryotic cell of claim 11.

**13.** A plant or animal cell of claim 12.

**14.** A plant which comprises the plant cell of claim 13.

**15.** A method for inactivating a target RNA in a cell which comprises contacting the target RNA within the cell with the compound of any of claims 1, 2, 3, 4, 5, or 6, wherein the compound is capable of interacting through base-pairing with the sequence of the target RNA under conditions such that the compound stably interacts through base-pairing with the target RNA and the target RNA is cleaved.

**16.** The method of claim 15, wherein the target RNA is an transcript of a gene which is endogenous to the cell.

**17.** The method of claim 15, wherein the target RNA is a transcript of a gene which is exogenous to the cell.

**18.** The method of any of claims 10, 15, 16, or 17, wherein the cell is a prokaryotic or eukaryotic cell.

**19.** The method of claim 18, wherein the cell is a plant or animal cell.

**20.** The method of claim 19, wherein the plant cell is a component of a plant.

**21.** Use of the compound of any of claims 1, 2, 3, 4, 5, or 6, or of the transfer vector of claim 9 in the manufacture of a medicament for the treatment of a condition associated with a target RNA in man or animals.

**23.** Use of the compound of any of claims 1, 2, 3, 4, 5, or 6 or of the transfer vector of claim 9 in the manufacture of a composition for the inactivation of a target RNA in plants.

**24.** Use of the compound of any of claim 6, or of a transfer vector which on transcription gives rise to such compound in the manufacture of a medicament for the treatment of a viral disease in man or animals.

**25.** Use of a ribozyme as a medicament for treatment of a disease in man or animals.

**26.** A method for inactivating a target RNA in a eukaryotic cell which comprises introducing into the eukaryotic cell an exogenous nucleic acid sequence which is, or is or on transcription gives rise to a ribozyme designed to cause specific cleavage of said target RNA at a site selected so as to cause the inactivation of said target RNA under conditions such that the target RNA is cleaved and inactivated.

**27.** The method of claim 26, wherein the ribozyme is formed in the eukaryotic cell.

**28.** The method of claim 26, wherein the ribozyme is formed outside the eukaryotic cell.

**29.** A eukaryotic host cell comprising an exogenous ribozyme designed to cause specific cleavage of a chosen endogenous or exogenous target RNA at a site selected so as to cause cleavage and inactivation of said target RNA.

**30.** Oligonucleotide capable of cleaving any chosen target RNA molecule, said oligonucleotide comprising (i) the catalytic region of a ribozyme, the catalytic activity of said region being independent of the sequence of the chosen target RNA, and (ii) a hybridizing region comprising a hybridizing arm extending from the 5' end of the catalytic region and a hybridizing arm extending from the 3' end of the catalytic region, said arms being of sufficient length to enable stable hybridization to regions of complementary sequence in the target RNA flanking the selected cleavage site.

Fig.Ia.

Fig.Ib.

Fig.Ic.

Fig.Id.

FIG.2a.

FIG.2b.

*FIG.3.*

5' CAT RNA TRANSCRIPT 3'

1      139 140            494 495      662 663       835

Rz$_{CAT}$-1         Rz$_{CAT}$-2     Rz$_{CAT}$-3

**Fig.4a.**

RzCAT-1 SITE:
#24

```
    Ala  Phe  Gln  Ser       Val  Ala  Gln
 5' G C A U U U C A G U C   A G U U G C U C A A 3'
    * * * * * * * *         * * * * * * * *
    U A A A G U C A         U C A A C G A G
 3'                 A         C              5'
                    A         U
                    G    A   G
                    C*G  G  A
                    A*U     U
                    G*C
                    G*C
                    A    G
                    G  U
```

**Fig.4b.**

RzCAT-2 SITE:

```
    #143
                          ↓
        Phe  Phe  Val     Ser  Ala  Asn      3'
 5'U G U U U U U C G U C   U C A G C C A A U C
      * * * * * * * *      * * * * * * * *
      A A A A A G C A      A G U C G G U U
   3'             A        C               5'
                  A        U
                  G    A     G
                   C*G  G  A
                   A*U     U
                   G*C
                   G*C
                  A    G
                   G  A
```

# FIG.4c.

RzCAT-3 SITE:

```
    #199
                          ↓
        Phe  His  Val     Gly  Arg  Met      3'
 5'GC U U C C A U G U C   G G C A G A A U G C
      * * * * * * * *      * * * * * * * *
      A A G G U A C A      C C G U C U U A
   3'             A        C               5'
                  A        U
                  G    A     G
                   C*G  G  A
                   A*U     U
                   G*C
                   G*C
                  A    G
                   G  U
```

# FIG.4d.

26

FIG.5a.

FIG.5b.

FIG.6.

EP 0 640 688 A1

RzCAT-1 CLEAVAGE
37oC, pH 8.0, 20mM Mg++

SUBSTRATE          3' PRODUCT

RzCAT-2 CLEAVAGE
37oC, pH 8.0, 20mM Mg++

SUBSTRATE          5' PRODUCT

RzCAT-3 CLEAVAGE
37oC, pH 8.0, 20mM Mg++

*FIG. 7.*

SUBSTRATE          5' PRODUCT

## Rz CAT-1 CLEAVAGE
### 50oC, pH 8.0, 20mM Mg++

% CLEAVED

MINUTES

—•— SUBSTRATE     —+— 3¹ PRODUCT

## Rz CAT-2 CLEAVAGE
### 50oC, pH 8.0, 20mM Mg++

% CLEAVED

MINUTES

—•—SUBSTRATE     —+— 5¹ PRODUCT

## Rz CAT-3 CLEAVAGE
### 50oC, pH 8.0, 20mM Mg++

% CLEAVED

MINUTES

*FIG.7.*(cont.)     —•—SUBSTRATE     —+— 5¹ PRODUCT

#139

5' Ile  Glu  Asn  Met  Phe  Phe  Val  │ Ser  Ala  Asn  Pro  Trp  Val  Ser  3'
  A U U G A G A A U A U G U U U U U C G U C  U C A G C C A A U C C C U G G G U G A G U
                          * * * *        * * * *
                          A G C A        A G U C
                    3'      A            C      5'
                            A            U
                            G        A   G
                            C*G    G   A
                            A*U        U
                            G*C
                            G*C
                            A        G
                            G  A          2×4

#139

5' Ile  Glu  Asn  Met  Phe  Phe  Val  │ Ser  Ala  Asn  Pro  Trp  Val  Ser  3'
  A U U G A G A A U A U G U U U U U C G U C  U C A G C C A A U C C C U G G G U G A G U
                    * * * * * * *    * * * * * * *
                    A A A A A G C A    A G U C G G U U
              3'                A      C            5'
                                A      U
                                G    A   G
                                C*G    G   A
                                A*U        U
                                G*C
                                G*C
                                A        G
                                G  A          2×8

*FIG.8a.*

#139

Ile Glu Asn Met Phe Phe Val ↓ Ser Ala Asn Pro Trp Val Ser

5' A U U G A G A A U A U G U U U U U C G U C U C A G C C A A U C C C U G G G U G A G U 3'

\* \* \* \* \* \* \* \* \* \* \*     \* \* \* \* \* \* \* \* \* \* \*

A U A C A A A A A G C A     A G U C G G U U A G G G A

3'     A     C     5'

A     U

G A G

C\*G G A

A\*U U

G\*C

G\*C

A G

G A

2×12

*Fig.8a. (cont.)*

EP 0 640 688 A1

FIG.9.

EP 0 640 688 A1

```
                    ↓
5'..AAGACCGUAAAGAAAAA..3'          TARGET SEQUENCE
     | | | | | | | |   | | | | | | | |
3'..UUCUGGCA UUCUUUUU..5'          RIBOZYME
            A  C
            A    U G
            G  A   G A
            C·G  G U
            A·U
            G·C
            G·C
            A  G
             G U
```

## Fig.10a.

```
                    ↓
5'..GAUAGUGUUCACCUUG..3'           TARGET SEQUENCE
     | | | | | | | |   | | | | | | | |
3'..CUAUCACA GUGGGAAC..5'          RIBOZYME
            A  C
            A    U G
            G    A   A
             C·G   G U
             A·U
             G·C
             G·C
             A   G
             G   U
```

## Fig.10b.

RIBOZYME CEV9x(+)    5'GAAGUCCUUCAG 3'

RIBOZYME CEV9x(-)    5'AGGGUCAGGUGA 3'

RIBOZYME CEV25x(+)    5'GAAGUCGAGGUC 3'

*FIG. 11.*

```
5'UCCCCGGGGAAACCUGGAGGAAGUC          GAGGUCGGGGACAGCUG 3'
  * * * * * * * * * * * * * * * * * * * * *    * * * * * * * * * * * * * * * * *
3'AGGGGCCCCUUUGGACCUCGUUCA          CUCCAGCCCCUGUCGAC 5'
                            A                  C
                            A                  U
                            G                  G
                              C     G G   A    G
                              A * U   U   A
                              G * C       U G
                              G * C
                              A       G
                              G U
```

*FIG. 12a.*

CLEAVAGE
PRODUCT ——→

    1       2

*FIG. 12b.*

5' Asn Met Phe Phe Val │ Ser Ala Asn Pro Trp 3'
A A U A U G U U U U U C G U C  U C A G C C A A U C C U G G
  * * * * * * * * * * *    * * * * * * * * * * *
  A U A C A A A A A G┌C A  ┌A G U C G G U U A G G G A
  3'                 │A    │C                      5'
                     │A    │U
                     │G   A  G
                     │C*G G   A
                     │A*U     U
                     │G*C
                     │G*C
                     │A    G
                     │G A

## Fig.13a.

5' Asn Met Phe Phe Val │ Ser Ala Asn Pro Trp 3'
A A U A U G U U U U U C G U C  U C A G C C A A U C C U G G
  * * * * * * * * * * *    * * * * * * * * * * *
  A U A C A A A A A G┌C A  ┌A G U C G G U U A G G G A
  3'                 │A    │C                      5'
                     │A    │U
                     │G   A  G
                     │C*G G   A
                     │A*U    Ⓒ
                     │G*C
                     │G*C
                     │A    C
                     │◁AAU

## Fig.13b.

37

Fig. 14a.

Fig. 14b.

```
        Asn   Met   Phe   Phe   Val  ¦  Ser   Ala   Asn   Pro   Trp
5'  A A U A U G U U U U U C G U C  ¦ U C A G C C A A U C C C U G G  3'
      * * * * * * * * * * *           * * * * * * * * * * * *
      A U A C A A A A A G C A         A G U C G G U U A G G G A
3'                      A                 C                      5'
                        A                 U
                        G       A       G
                          C*G     G   A
                          A*U         U
                          G*C
                          G*C
                        A       G
                        G A
```

*FIG.15a.*

```
        Asn   Met   Phe   Phe   Val  ¦  Ser   Ala   Asn   Pro   Trp
5'  A A U A U G U U U U U C G U C  ¦ U C A G C C A A U C C C U G G  3'
      * * * * * * * * * * *           * * * * * * * * * * * *
      A U A C A A A A A G C A         A G U C G G U U A G G G A
3'                      A                 C                      5'
                        A                 U
                        G       A       G
                          C*G     G   A
                          A*U         U
                          G*C
                          G*C
                          C*G
                          A*U
                          G*C
                          G*C
                        A       G
                        G A
```

*FIG.15b.*

FIG.16.

FIG.17.

European Patent
Office

PARTIAL EUROPEAN SEARCH REPORT

Application Number

which under Rule 45 of the European Patent Convention EP 94 10 7862
shall be considered, for the purposes of subsequent
proceedings, as the European search report

## DOCUMENTS CONSIDERED TO BE RELEVANT

| Category | Citation of document with indication, where appropriate, of relevant passages | Relevant to claim | CLASSIFICATION OF THE APPLICATION (Int.Cl.4) |
|---|---|---|---|
| A | NATURE, vol.328, 13 August 1987, LONDON GB pages 596 - 600 UHLENBECK, O.C. 'A small catalytic oligoribonucleotide' * the whole article, and especially figure 1a * --- | 1-4,6,8, 9 | C12N15/11 C12N9/00 C07H21/02 A61K31/70 |
| D,A | CELL, vol.50, 3 July 1987, CAMBRIDGE, NA US pages 9 - 16 FORSTER, A. & SYMONS, R. 'Self-cleavage of virusoid RNA is performed by the proposed 55-nucleotide active site' * the whole article, and especially figure 8 * --- | 1 | |
| D,A | CELL, vol.49, 24 April 1987, CAMBRIDGE, NA US pages 211 - 220 FORSTER, A. & SYMONS, R. 'Self-cleavage of plus and minus RNAs of a virusoid and a structural model for the active sites' --- -/-- | | |

TECHNICAL FIELDS
SEARCHED     (Int.Cl.4)

C12N
A61K

## INCOMPLETE SEARCH

The Search Division considers that the present European patent application does not comply with
the provisions of the European Patent Convention to such an extent that it is not possible to carry
out a meaningful search into the state of the art on the basis of some of the claims
Claims searched completely :
Claims searched incompletely :
Claims not searched :
Reason for the limitation of the search:

see sheet C

| Place of search | Date of completion of the search | Examiner |
|---|---|---|
| THE HAGUE | 30 November 1994 | Andres, S |

EPO FORM 1503 03.82 (P04C07)

| | DOCUMENTS CONSIDERED TO BE RELEVANT | | CLASSIFICATION OF THE APPLICATION (Int.Cl.4) |
|---|---|---|---|
| Category | Citation of document with indication, where appropriate, of relevant passages | Relevant to claim | |
| P,X | NATURE, vol.334, 18 August 1988, LONDON GB pages 585 - 591 HASELOFF, J. & GERLACH, W. 'Simple RNA enzymes with new and highly specific endoribonuclease activities' * page 587, right column, line 23 - page 591 * --- | 1-4,6,8, 9,15-17, 30 | |
| P,X | NATURE, vol.334, 21 July 1988, LONDON GB pages 196 - 197 WALBOT, V. & BRUENING, G. 'Plant development and ribozymes for pathogens' * page 197 * ----- | 1-4 | TECHNICAL FIELDS SEARCHED (Int.Cl.4) |